# EUROPÄISCHE PATENTSCHRIFT

(11) **EP 2 833 948 B1**
(45) Veröffentlichungstag und Bekanntmachung des Hinweises auf die Patenterteilung: **18.05.2016**
(21) Anmeldenummer: 13709942.0
(22) Anmeldetag: 19.03.2013
(51) Int. Cl.: A61M 5/31, A61M 5/34

(54) **VERSCHLUSSSYSTEM FÜR EINEN APPLIKATIONSEINRICHTUNGSANSATZ AN EINEM BEHÄLTER FÜR PHARMAZEUTISCHE PRÄPARATE, UND VERWENDUNG, BEHÄLTER MIT VERSCHLUSSSYSTEM, SPRITZE FÜR MEDIZINISCHE ZWECKE UND VERFAHREN ZUR MONTAGE**
CLOSURE SYSTEM FOR AN APPLICATOR PROJECTION ON A CONTAINER FOR PHARMACEUTICAL PREPARATIONS, USE, CONTAINER WITH CLOSURE SYSTEM, SPRAY FOR MEDICAL PURPOSES, AND ASSEMBLY METHOD
SYSTÈME DE FERMETURE POUR PIÈCE RAPPORTÉE DE DISPOSITIF D'APPLICATION SUR UN CONTENANT POUR PRÉPARATIONS PHARMACEUTIQUES, ET UTILISATION, CONTENANT DOTÉ D'UN SYSTÈME DE FERMETURE, SERINGUE À USAGE MÉDICAL ET PROCÉDÉ DE MONTAGE

(30) Priorität: 04.04.2012 DE 102012006885; 04.04.2012 DE 102012006886
(43) Veröffentlichungstag der Anmeldung: 11.02.2015
(73) Patentinhaber: Boehringer Ingelheim International GmbH, 55216 Ingelheim (DE)
(72) Erfinder: HAEFELE, Friedrich, 55216 Ingelheim (DE); HEMMINGER, Markus, 55216 Ingelheim (DE); SACHSSE, Tobias, 55216 Ingelheim (DE); BOJE, Klaus, 55216 Ingelheim (DE)
(74) Vertreter: Höfer, Friederike
(86) Internationale Anmeldenummer: PCT/EP2013/055668
(87) Internationale Veröffentlichungsnummer: WO 2013/149821

(56) Entgegenhaltungen:
- EP-A1- 0 397 951
- EP-A2- 0 749 760
- WO-A2-2004/045681
- DE-A1-102008 013 198
- DE-C1- 19 537 163

## Beschreibung

Die Erfindung betrifft ein Verschlusssystem für einen Applikationseinrichtungs-Ansatz an einem Behälter für pharmazeutische Präparate und eine Verwendung des Verschlusssystems. Die Erfindung betrifft ferner einen Behälter für pharmazeutische Präparate mit einem Verschlusssystem. Die Erfindung betrifft des Weiteren eine Spritze für medizinische Zwecke mit einem Behälter und einem Verschlusssystem und ein Verfahren zur Montage.

Eine Spritze für medizinische Zwecke ist beispielsweise aus der Druckschrift EP 0 397 951 A1 und DE 195 37 163 C1 vorbekannt. Diese umfasst einen, an einem Mundstück des Behälters angesetzten, durch ein Verschlusselement in Form von beispielsweise einem Tip-Cap bis zum Gebrauch dessen verschlossenen Applikationseinrichtungs-Ansatz, welcher zum Anbringen einer Applikationseinrichtung, beispielsweise einer Kanüle ausgeführt ist. Der Applikationseinrichtungs-Ansatz ist an einem Teilbereich der Überwurfkappe ausgebildet und wird über diese am Mundstück gehalten und dicht mit dem Behälter verbunden. Die Überwurfkappe wird durch einen aufgeschobenen und an ihr einrastenden Sicherungsring unlösbar am zylindrischen Mundstück des Behälters fixiert, wobei der Sicherungsring eine mit diesem fest verbundene, aber abtrennbar ausgebildete Sicherungskappe trägt, die den Applikationseinrichtungs-Ansatz sowie das Verschlusselement umschließt und dazu aus einem im Wesentlichen hohlzylindrischen Körper besteht, der an einem vom Behälter abgewandten Endbereich zumindest teilweise durch eine Stirnwand verschlossen ist. Die Druckschrift DE 195 37 163 C1 offenbart eine Weiterentwicklung der zumindest den Endbereich des Applikationseinrichtungs-Ansatzes und das Verschlusselement umschließenden Verschluss-Sicherungseinheit zur Realisierung eines Orginalitätsverschlusses, bei welcher neben den, die Sollbruchstelle bildenden Verbindungsstegen zwischen Sicherungskappe und Sicherungsring zusätzlich noch Abstandshalter vorgesehen sind, die in Abständen zwischen zwei benachbarten Stegelementen zwischen Sicherungskappe und Sicherungsring vorgesehen sind. Das durch das Verschlusselement, den Applikationseinrichtungs-Ansatz und die Verschluss-Sicherungseinheit charakterisierte Verschlusssystem ermöglicht einen druckdichten, mediendichten und mikrobiell dichten Verschluss des mindestens ein pharmazeutisches Präparat beinhaltenden Behälters zu Lagerungszwecken. Erst bei gewünschtem Gebrauch wird die Verbindung zwischen Sicherungskappe und Sicherungsring zerstört, das Verschlusselement aus seiner den Applikationseinrichtungs-Ansatz dichtenden Position verbracht und die Verbindung zum Innenraum des Behälters freigegeben. Zur Montage der Spritze ist es erforderlich, die einzelnen Bestandteile des Verschlusssystems zunächst auf entsprechend dafür ausgelegten Maschinen zu fertigen, falls erforderlich zu reinigen, und gegebenenfalls zu silikonisieren und zu sterilisieren, z.B. durch autoklavieren, um diese dann unter strengen Reinraumbedingungen dem weiteren Herstellungsprozess zuzuführen. Zur Verbesserung der Verfahrensabläufe ist aus der Druckschrift EP 1 034 810 A1 ein Verschlusssystem vorbekannt, bei welchem der Sicherungsring mit einer Sicherungskappe abtrennbar über Verbindungsmittel verbunden ist und das Verschlusselement formschlüssig in der Sicherungskappe gehalten wird. Das Verschlusssystem kann als vollständig vormontierte und behandelte Einheit bei der Montage einer Spritze gehandhabt werden. Es bestehen jedoch sehr hohe Anforderungen an die Fertigungsgenauigkeit, um im verschlossenen Zustand des Applikationseinrichtungs-Ansatzes die Dichtheit zu gewährleisten.

Es gibt pharmazeutische Präparate, insbesondere Arzneimittel, die im flüssigen Zustand sehr schnell ihre Wirksamkeit verlieren. Um diese trotz ihrer kurzen Haltbarkeit verwenden zu können, wurden spezielle Vorrichtungen und Verfahren zur Lyophilisation entwickelt. So lassen sich pharmazeutische Präparate, die nicht über längere Zeit in Lösung verwendet werden können, durch Lyophilisieren haltbar machen und gegebenenfalls unter Luftausschluss aufbewahren. Die Trockensubstanz wird erst unmittelbar vor Gebrauch wieder gelöst, d.h. rekonstituiert. Hierzu sind Zweikomponenten-Systeme bekannt geworden, um das Lyophilisat unmittelbar vor Gebrauch wieder zu lösen. Die Aufbewahrung erfolgt in Einkammer-Behältern mit integriertem Doppelkammer-System oder Doppelkammer-Behältern, die an einem Endbereich mit dem aus dem Stand der Technik bekannten Verschlusssystem verschließbar sind. Da nach Abheben des Verschlusselementes bei Entlüftung des Behälters und während der Rekonstitution ein Herausspritzen von Lösung möglich ist, wird für diesen Vorgang in der Regel eine zusätzliche Kappe als Spritzschutz verwendet. Das Aufsetzen der Kappe ist aufwendig und gewährleistet weder eine vollständige Unterbindung der Gefahr einer Kontamination der Umgebung mit der Lösung noch einer Kontamination der Lösung durch die Umgebung. Ferner wird die vollständige Sterilität der Lösung gefährdet.

Ein Behälter mit einem gattungsbildenden Verschlusssystem gemäß dem Oberbegriff aus Anspruch 1 ist aus den Druckschriften DE 195 37 163 C1 und DE 41 27 650 A1 vorbekannt. Diese Ausführungen ermöglichen zwar einen sterilen Verschluss noch im Lyophilisator, allerdings ist ein Spritzschutz beim Öffnen nicht gegeben.

Demgegenüber sind aus den Druckschriften US 5,624,402 und DE 44 34 644 C2 Verschlusssysteme vorbekannt, die beim Öffnen des Verschlusselementes zwar einen Spritzschutz gewährleisten, jedoch nicht geeignet sind, einen Behälter, insbesondere Doppelkammerbehälter noch im Lyophilisator zu verschließen.

Der Erfindung liegt die Aufgabe zugrunde, ein Verschlusssystem für einen Applikationseinrichtungs-Ansatz an einem Behälter für pharmazeutische Präparate derart weiterzuentwickeln, dass dieses geeignet ist, zum Einen einen Behälter mit Doppelkammersystem, insbesondere Doppelkammer-Behälter auf einfache und schnelle Art und Weise in einem Lyophilisator zu verschließen und zum Anderen beim gewünschten Anschluss einer Applikationseinrichtung an einen Applikationseinrichtungs-Ansatz die Sterilität bei der erforderlichen Entlüftung des angeschlossenen Behälters sicher zu gewährleisten, wobei sowohl die Gefahr einer Kontamination durch herausspritzendes Präparat oder Lösung als auch eine Kontamination der Lösung vermieden wird. Des Weiteren soll ein Orginalitätsverschluss ein missbräuchliches Öffnen vor Gebrauch vermeiden.

Die erfindungsgemäße Lösung ist durch die Merkmale der Ansprüche 1, 21, 22, 24 und 25 charakterisiert. Vorteilhafte Weiterbildungen und Ausgestaltungen sind in den Unteransprüchen wiedergegeben.

Ein Verschlusssystem für einen sich entlang einer theoretischen Längsachse erstreckenden und eine Durchgangsöffnung aufweisenden Applikationseinrichtungs-Ansatz an einem Behälter für pharmazeutische Präparate geeignet zum Anschluss einer Applikationseinrichtung, umfassend
- ein Verschlusselement mit zumindest einem, eine Dichtfläche aufweisenden Bereich zur Abdichtung der Durchgangsöffnung des Applikationseinrichtungs-Ansatzes,
- ein hülsenartiges Anschlusselement zur kraft- und/oder formschlüssigen Verbindung mit dem Behälter im applikationseinrichtungsseitigen Endbereich;
- eine koaxial zum Anschlusselement angeordnete und dieses in Umfangsrichtung umschließende Verschluss-Sicherungseinheit mit einem Sicherungsring und einem mit dem Sicherungsring abtrennbar über Verbindungsmittel unter irreversibler Zerstörung dieser verbundenes Sicherungsteil, wobei die Verschluss-Sicherungseinheit ausgelegt ist, zumindest den Endbereich des Applikationseinrichtungs-Ansatzes und das Verschlusselement in Umfangsrichtung um die Längsachse über einen Teilbereich der Erstreckung in Längsrichtung zu umschließen und das hülsenartige Anschlusselement am Behälter in Umfangsrichtung durch Relativbewegung gegenüber dem Anschlusselement in Längsrichtung von einer ersten in eine zweite Montagestellung zu umschließen und vorzugsweise zu fixieren, ist erfindungsgemäß dadurch gekennzeichnet, dass das Verschlusselement wenigstens mittelbar mit einem vom Applikationseinrichtungs-Ansatz oder einem diesen tragenden Funktionselement oder dem Sicherungsring gebildeten Anschlussteil lösbar kraft- und/oder formschlüssig verbunden ist. Dabei verschließt das Verschlusselement in einer als Verschluss-Stellung definierten ersten Funktionsstellung die Durchgangsöffnung am Appiikationseinrichtungs-Ansatz druckdicht, mediendicht und mikrobiell dicht. Die form- und/oder kraftschlüssige Verbindung ist derart angeordnet und ausgeführt, geeignet zu sein, nach Abtrennung des Sicherungsteils vom Sicherungsring eine Relativbewegung zwischen den an der Verbindung beteiligten Bauteilen, insbesondere Verschlusselement und Anschlussteil, mit einer Richtungskomponente in Richtung der Längsachse aus der ersten Funktionsstellung (Verschluss-Stellung) in zumindest eine weitere Funktionsstellung des Verschlusselementes (Spritzschutz- und/oder Entlüftungsstellung) unter zumindest teilweiser Aufrechterhaltung dieser Verbindung zu ermöglichen, wobei in der zumindest einen weiteren Funktionsstellung die druck- , medien- und mikrobiell dichte Abdichtung der Durchgangsöffnung am Applikationseinrichtungs-Ansatz durch das Verschlusselement unter Bildung eines zwischen Verschlusselement, Applikationseinrichtungs-Ansatz und/oder Anschlussteil gebildeten Zwischenraumes aufgehoben ist.

Die Längsachse beschreibt die Ausrichtung des Verschlusssystems und der einzelnen Teile dieser in Längsrichtung. Diese fällt bei vorteilhafter zylindrischer Ausgestaltung und koaxialer Anordnung der einzelnen Bestandteile mit der Mittenachse zusammen.

Mediendicht beinhaltet die Dichtheit jeweils gegenüber zumindest einem Medium oder mehreren der nachfolgenden genannten Medien oder Gemischen aus diesen: Flüssigkeiten, Gasen, Feststoffen oder Gemischen aus diesen.

Unter einer wenigstens mittelbaren Verbindung zwischen zwei Bauteilen wird eine Verbindung verstanden, welche entweder direkt zwischen beiden Bauteilen ausgeführt ist oder aber indirekt, d.h. über weitere zwischengeordnete Übertragungselemente beziehungsweise Bauteile realisiert wird. Eine derartig mittelbare Verbindung wird beispielsweise zwischen einem das Verschlusselement tragenden Bauteil in Form eines Aufnahmeteils und einem Anschlussteil ausgeführt. Vorzugsweise erfolgt die Verbindung des Verschlusselementes beziehungsweise eines dieses aufnehmenden Aufnahmeteils direkt mit einem Anschlussteil. Denkbar sind jedoch auch Verbindungen mit einem mit dem Anschlussteil verbundenen Bauteil.

Als Anschlussteil können verschiedene Bestandteile des Verschlusssystems fungieren. Vorzugsweise wird das Anschlussteil vom Sicherungsring der Verschluss-Sicherungseinheit gebildet. Denkbar ist auch die Nutzung eines anderen Funktionselementes, beispielsweise des Applikationseinrichtungs-Ansatzes oder eines diesen tragenden Bauteiles oder einer Überwurfkappe.

Die Funktionsstellung des Verschlusselementes beschreibt dessen Lage, insbesondere die Anordnung der zumindest einen Dichtfläche gegenüber der Durchgangsöffnung des Applikationseinrichtungs-Ansatzes zumindest im montierten Zustand des Verschlusssystems am Behälter. Im Funktionszustand des Applikationseinrichtungs-Ansatzes "druck-, medien- und mikrobiell dicht verschlossen" entspricht die Funktionsstellung des Verschlusselementes der ersten Funktionsstellung, in welcher die Durchgangsöffnung des Applikationseinrichtungs-Ansatzes über dieses verschlossen ist. Die zweite Funktionsstellung kann fest definiert, beispielsweise durch eine Raststellung oder beliebig innerhalb des theoretisch möglichen Verbindungsbereiches in Richtung der Längsachse der zumindest mittelbaren Verbindung zwischen Verschlusselement und Anschlussteil einstellbar sein. Im Funktionszustand des Applikationseinrichtungs-Ansatzes "nicht verschlossen beziehungsweise Anschluss der Applikationseinrichtung" ist das Verschlusselement vollständig von diesem entfernt.

Der Begriff "Montagestellung" beschreibt die möglichen Positionen der Verschluss-Sicherungseinheit und der einzelnen Komponenten der Verschluss-Sicherungseinheit zueinander gegenüber dem Anschlusselement zur Fixierung am Behälter während des Verschließvorganges des Behälters. Grundlegend sind zumindest zwei Montagestellungen definiert, denkbar sind jedoch auch Zwischenstellungen.

Die erfindungsgemäße Lösung bietet den Vorteil einer einfach realisierbaren Integration der Spritzschutz- und Entlüftungsfunktion in ein ohnehin erforderliches Verschlusssystem für einen Applikationseinrichtungs-Ansatz durch Ausnutzung der Bestandteile des Verschlusssystems, wodurch eine hohe Funktionskonzentration bei geringer Bauteilanzahl gegeben ist. Im Einzelnen bilden die den Zwischenraum in der zweiten Funktionsstellung des Verschlusselementes begrenzenden Bestandteile des Verschlusssystems gleichzeitig den Spritzschutz. Eine zusätzlich vorzusehende Spritzschutzkappe, die nach Entfernung des Verschlusselementes angebracht wird, und die damit verbundenen Nachteile werden vermieden. Ferner kann bereits durch die Auslegung des sich ergebenden Zwischenraumes in der zweiten Funktionsstellung auch bei dichter Ausführung der Verbindung ein Druckabbau in den Zwischenraum erfolgen. Die erfindungsgemäße Ausführung ist ferner in besonders vorteilhafter Weise für den Verschluss eines Behälters für pharmazeutische Präparate mit Doppelkammersystem, insbesondere einen Doppelkammerbehälter im Lyophilisator geeignet, da aufgrund der durch die Verbindung zwischen Verschlusselement und Anschlussteil bereits fest definierten Lage des Verschlusselementes im Verschlusssystem, die koaxiale Anordnung von Verschlusselement, Verschluss-Sicherungseinheit und Anschlusselement zueinander und die Möglichkeit der Relativbewegung zwischen Verschluss-Sicherungseinheit und Anschlusselement in Längsrichtung zwischen einer ersten und zweiten Montagestellung nur noch eine Kraft in Längsrichtung zum Verschließen benötigt wird, die innerhalb des Lyophilisators erzeugbar ist.

In einer vorteilhaften Weiterbildung sind Entlüftungsmittel vorgesehen, die derart angeordnet und ausgeführt sind, geeignet zu sein, zumindest in der zweiten Funktionsstellung des Verschlusselementes die Durchgangsöffnung am Applikationseinrichtungs-Ansatz mit der Umgebung zu verbinden. Dadurch kann ein vollständiger Druckausgleich mit der Umgebung frei von austretendem Medium aus dem angeschlossenen Behälter erfolgen. Gemäß einer besonders vorteilhaften Ausführung sind die Entlüftungsmittel zumindest teilweise in der zumindest mittelbaren Verbindung zwischen Verschlusselement und Anschlussteil integriert. In Abhängigkeit der Anordnung und Ausführung der Entlüftungsmittel ist die Entlüftung damit gezielt einstellbar.

Die Entlüftungsmittel können dazu in einer ersten Ausbildung wenigstens teilweise von den Verbindungsmitteln der zumindest mittelbaren Verbindung zwischen Verschlusselement und Anschlussteil gebildet werden und/oder in einer weiteren zweiten Ausbildung zumindest einen separaten Entlüftungskanal umfassen. Beide Ausbildungen sind auch miteinander kombinierbar.

In der Ausbildung des Verschlusssystems frei von Bauteilen mit integriertem Entlüftungskanal wird die Entlüftungsstellung allein durch zumindest teilweises Aufheben der zumindest mittelbaren Verbindung zwischen dem Verschlusselement und dem Anschlussteil durch Relativbewegung dieser zueinander in die geeignete Entlüftungsstellung erreicht.

Bei einer Ausbildung mit mindestens einem Entlüftungskanal ist dieser Kanal/sind diese Kanäle entweder in die Verbindungsmittel an einem der miteinander zu verbindenden Bauteile, beispielsweise durch Vorsehen randoffener Aussparungen, oder durch Ausbildung von Verbindungsmitteln freier Bereiche an zumindest einem der miteinander zu verbindenden Bauteile, eingearbeitet.

Bei allen vorgenannten Ausbildungen kann die Spritzschutz- und/oder Entlüftungsstellung für den Nutzer erkennbar ausgeführt sein, vorzugsweise visuell oder auch ertastbar, beispielsweise über entsprechende Markierungen, die am Verschlusselement und dem Anschlussteil derart angeordnet und ausgeführt sind, geeignet zu sein, bei Erreichen der zweiten Funktionsstellung (Spritzschutz- und/oder Entlüftungsstellung) eine vordefinierte Stellung zueinander einzunehmen. Vorzugsweise werden Markierungen gewählt, die in der zweiten Funktionsstellung eine Lage zueinander einander gegenüberstehend oder überlappend einnehmen.

Eine wenigstens mittelbare Verbindung zwischen Verschlusselement und Anschlussteil kann gemäß einer ersten Ausführung indirekt, d.h. über zumindest ein weiteres mit dem Verschlusselement verbundenes Bauteil oder gemäß einer zweiten Ausführung direkt erfolgen. Dabei wird in der ersten Ausführung die zumindest mittelbare Verbindung zwischen Verschlusselement und Anschlussteil indirekt von einer Verbindung zwischen einem, das Verschlusselement zumindest in Umfangsrichtung um die Längsachse betrachtet umschließenden und mit diesem verbundenen Aufnahmeteil gebildet, und die Verbindung zwischen Verschlusselement und Aufnahmeteil als eine der nachfolgend genannten Verbindungen oder einer Kombination aus diesen ausgeführt:
- kraftschlüssige Verbindung
- formschlüssige Verbindung
- stoffschlüssige Verbindung
Der in Richtung der Längsachse und zumindest entgegen der Bewegungsrichtung des Verschlusselementes beim Lösen wirksame, zumindest mittelbar zwischen Verschlusselement und Sicherungsteil gebildete Formschluss wird dann zwischen Aufnahmeteil und Sicherungsteil gebildet. Diese Lösung bietet den Vorteil einer geometrisch einfachen und kompakten Ausgestaltung des Verschlusselementes als Vollelement. Die Materialwahl für das Anschlussteil, insbesondere im Verbindungsbereich ist nicht durch die für die Dichtfunktion zulässigen Materialen des Verschlusselementes beschränkt. Vielmehr kann hinsichtlich des Aufnahmeteils und des Anschlussteils auf kostengünstige Materialien und einfache Herstellungsverfahren zurückgegriffen werden. Die Integration des Verschlusselementes in das Aufnahmeteil bietet ferner den Vorteil der Ausbildung eines in Längsrichtung entsprechend großen Handhabungsbereiches beim Öffnen des Verschlusssystems. In diesem können speziell geformte und/oder ausgebildete Angriffsbereiche vorgesehen, insbesondere eingearbeitet werden.

Eine kraftschlüssige Verbindung zwischen Verschlusselement und Aufnahmeteil kann beispielsweise durch eine Pressverbindung realisiert werden. Dabei wird das Verschlusselement mit Übermaß in Umfangsrichtung gegenüber dem Innenumfang des dieses aufnehmenden Aufnahmeteils ausgeführt. Die Auslegung der Pressverbindung erfolgt vorzugsweise derart, dass die in der Funktionsstellung "verschlossen" zur Aufrechterhaltung der Verbindung erforderlichen Kräfte von dieser aufnehmbar sind. In einer vorteilhaften Weiterbildung dieser Verbindungsvariante ist das Aufnahmeteil im in Einbaulage vom Applikationseinrichtungs-Ansatz weggerichteten Endbereich zumindest teilweise geschlossen beziehungsweise unter Ausbildung einer entgegen der Bewegungsrichtung in Längsrichtung beim Lösen ausgerichteten Anlagefläche für das Verschlusselement zur Erzeugung einer bei Realisierung der Verbindung zwischen Aufnahmeteil und Anschlussteil auf das Verschlusselement ausgeübten Vorspannkraft ausgestaltet, wodurch das Verschlusselement sicher im Aufnahmeteil gehalten wird. Im erst genannten Fall kann das Aufnahmeteil einseitig zumindest teilweise geschlossen oder als Kappe ausgeführt sein. Im zweiten Fall kann am Innenumfang des geschlossenen Endbereiches des Aufnahmeteils ein von diesem weggerichteter Vorsprung unter Ausbildung einer Anlagefläche ausgebildet sein.

In einer weiteren Variante ist das Verschlusselement mit dem Aufnahmeteil formschlüssig verbunden. Die Verbindung kann beispielsweise über eine Rastverbindung erzeugt werden. Die Formschlussmittel, insbesondere Rastmittel können sich dabei jeweils entweder über einen Teilbereich in Umfangsrichtung um die Längsachse erstreckend ausgeführt sein oder aber vorzugsweise vollständig, wodurch eine gleichmäßige Kraftverteilung realisierbar ist.

In einer zweiten Ausführung ist das Verschlusselement direkt mit dem Anschlussteil verbunden.

Um ein mehrmaliges Öffnen und Verschließen des Applikationseinrichtungs-Ansatzes ausschließen zu können, ist eine irreversible abtrennbare Verbindung zwischen Sicherungsring und Sicherungsteil vorgesehen. Dieser sogenannte Orginalitätsverschluss wird in einer ersten Variante durch die Funktionskonzentration von Entsicherungsfunktion und Verbringen in die zweite Funktionsstellung realisiert. Das Verschlusselement beziehungsweise das dieses tragende Aufnahmeteil ist über einen in Richtung der Längsachse wirksamen, insbesondere zumindest entgegen der Bewegungsrichtung beim Lösen des Verschlusselementes ausgerichteten, zumindest mittelbar zwischen Verschlusselement und Sicherungsteil gebildeten Formschluss gegenüber dem Sicherungsteil fixiert. Dieser Formschluss ist vorzugsweise im Verbindungsbereich zwischen Sicherungsteil und Sicherungsring angeordnet, wodurch bei Aufhebung der Verbindung zwischen Sicherungsteil und Sicherungsring zwangsweise auch der Formschluss aufgehoben wird. Entsicherung und Einstellung der zweiten Funktionsstellung sind somit zwangsgekoppelt, wodurch ein Handhabungsschritt bei gewünschtem Anschluss einer Applikationseinrichtung entfallen kann. Diese Ausführung ist durch eine hohe Funktionskonzentration mit minimaler Bauteilanzahl charakterisiert.

Zur Realisierung der Zwangskopplung zwischen der Trennung der Verbindung zwischen Sicherungsring und Sicherungsteil und Lösung des zumindest in Längsrichtung entgegen der Löserichtung des Verschlusselementes wirkenden Formschlusses zwischen Verschlusselement beziehungsweise Aufnahmeteil und Sicherungsteil werden zur Ausbildung des Formschlusses Bestandteile des abzutrennenden Sicherungsteils genutzt. Der Formschluss kann dabei direkt im Verbindungsbereich zwischen Sicherungsring und Sicherungsteil angeordnet sein oder aber versetzt zu diesem.

In einer besonders vorteilhaften Ausbildung können Sicherungsteil und Sicherungsring über wenigstens ein in Längsrichtung ausgebildetes Verbindungsmittel, beispielsweise einen Steg mit in Längsrichtung unveränderlichem oder veränderlichem Querschnitt miteinander verbunden sein, wobei das Sicherungsteil eine zum Sicherungsring weisende und zu diesem beabstandete Fläche aufweist, die als Anschlagfläche in Längsrichtung für zumindest einen am Verschlusselement oder dem Aufnahmeteil in Umfangsrichtung um diesen ausgebildeten zumindest ringsegmentförmigen elastischen Sicherungsvorsprung in der ersten Funktionsstellung des Verschlusselementes fungiert. Dabei können ein- oder mehrere derartige Sicherungsvorsprünge vorgesehen werden, die sich jedoch allein oder gemeinsam über einen Mindestwinkelbereich in Umfangsrichtung um das Verschlusselement oder das Aufnahmeteil erstrecken müssen.

In einer zweiten Variante wird der Orginalitätsverschluss durch Verbindungsstege zwischen Sicherungsteil und Sicherungsring gebildet.

Vorzugsweise ist die wenigstens mittelbare, eine Relativbewegung zwischen Verschlusselement oder Aufnahmeteil und Anschlussteil zulassende Verbindung zumindest über einen Teilbereich mit einer in Richtung der Längsachse der Bewegungsrichtung zum Lösen des Verschlusselementes entgegengerichtet wirkenden Form- und/oder Kraftschlussverbindung ausgeführt. Durch den entgegen der Bewegungsrichtung beim Lösen ausgebildeten kraft- und/oder formschlüssigen Teilbereich des Verbindungsbereiches wird quasi ein selbsttätiges Verschieben der an der Verbindung beteiligten Bauteile unter Aufhebung der Abdichtung sicher ausgeschlossen und über die Verbindung das Verschlusselement mit einer Grundpresskraft gegenüber dem Applikationseinrichtungs-Ansatz gehalten. In Abhängigkeit der gewählten Verbindungsart ist dieses Merkmal der Verbindung bereits inhärent, beispielsweise bei einer Schraubverbindung, bei welcher bei Haftreibung zwischen den Gewindegängen miteinander zu verbindender Bauteile keine Relativbewegung erfolgt.

Bezüglich der Ausführung der eine Relativbewegung zwischen Verschlusselement oder Aufnahmeteil und Anschlussteil zulassenden Verbindung besteht eine Mehrzahl von Möglichkeiten. Allen gemeinsam ist, dass die Verbindung bei der Relativbewegung innerhalb dieser eine Bewegung zumindest eines der Bauteile in Richtung der Längsachse ermöglicht. Bei der Relativbewegung kann es sich um Bewegungen in nur einer Richtung oder eine überlagerte Bewegung, beispielsweise einer Umdrehung in Umfangsrichtung unter gleichzeitiger Bewegung in Längsrichtung handeln. Ferner kann ein Bauteil gegenüber einem Referenzbauteil oder einer Referenzfläche in seiner Lage unveränderlich und das andere Bauteil gegenüber diesem bewegbar sein. Denkbar ist auch die Bewegung beider verbundenen Bauteile gegeneinander.

In einem ersten Lösungsansatz finden für die die Relativbewegung zulassende Verbindung kraftschlüssige Verbindungen Verwendung, wie beispielsweise Schraubverbindungen. In einem zweiten Lösungsansatz sind formschlüssige Verbindungen einsetzbar. Ein dritter Lösungsansatze beinhaltet ein kombiniertes Verbindungssystem aus beiden vorgenannten.

In einer vorteilhaften Variante gemäß dem ersten Lösungsansatz ist die eine Relativbewegung zwischen dem Verschlusselement oder Aufnahmeteil und dem Anschlusselement mit einer Richtungskomponente in Richtung der Längsachse zulassenden Verbindung von einer Schraubverbindung gebildet, umfassend einen ein Innengewinde tragenden Teilbereich an einem der miteinander zu verbindenden Bauteile - - Verschlusselement beziehungsweise ein dieses tragendes Aufnahmeteil oder Anschlussteil - und einen ein Außengewinde tragenden Teilbereich am jeweils anderen der miteinander zu verbindenden Bauteile - Anschlussteil oder Verschlusselement beziehungsweise ein dieses tragendes Aufnahmeteil.

In einer vorteilhaften Weiterbildung dieser Variante des ersten Lösungsansatzes ist entweder kein oder wenigstens ein, sich mit zumindest einer Hauptrichtungskomponente parallel zur Längsachse verlaufender Entlüftungskanal in der Schraubverbindung vorgesehen. Der Vorteil des integrierten Entlüftungskanals besteht in der einfachen Herstellung entweder bereits bei der Fertigung des Gewindes oder der nachträglichen Einarbeitung in dieses. Bei der Lösung mit mindestens einem Entlüftungskanal ist dieser Kanal/sind diese Kanäle entweder in das Gewinde eingearbeitet, beispielsweise durch Vorsehen randoffener Aussparungen an den Gewindegängen zumindest an einem der zu verbindenden Bauteile, oder durch Unterbrechung der Gewindegänge unter Ausbildung von einem Gewinde freier Bereiche an zumindest einem der miteinander zu verbindenden Bauteile - Verschlusselement beziehungsweise Aufnahmeteil oder Anschlussteil -, wobei die Anordnung und Dimensionierung der von einem Gewinde freien Bereiche derart erfolgt, dass der einzelne zwischen zwei von einem Gewinde freien Bereichen angeordnete Gewinde tragende Bereich einen Winkelbereich umfasst, der aufsummiert über die gewindetragenden Bereiche eine einwandfreie Funktionalität der Schraubverbindung gewährleistet.

Der Entlüftungskanal wird in vorteilhafter Weise durch Unterbrechung des Gewindes an einem der beiden Bauteile oder beiden erzeugt, beispielsweise indem lediglich Gewindegangsegmente in Umfangsrichtung am entsprechenden Innenumfang- oder Außenumfang bildenden Teilbereich vorgesehen werden. Ein derart vorteilhafter aufsummierter Winkelbereich beträgt beispielsweise zwischen jeweils einschließlich 5° bis 90°, vorzugsweise 8° bis 60°, besonders bevorzugt 10° und 45° bezogen auf den vollen Umfang 360°. Der beanspruchte Winkelbereich gewährleistet noch einen sicheren Eingriff der miteinander zu verbindenden Bauteile und die Funktion der Verbindung.

In einer vorteilhaften Weiterbildung wird die Funktionsstellung (Spritzschutz- und/oder Entlüftungsstellung) zwischen Verschlusselement und Applikationseinrichtungs-Ansatz als Funktion der Geometrie und Dimensionierung der Gewinde erreicht. Um dies zu erzielen kann zum Beispiel die Gewindesteigung und die Dicke der Gewindegänge angepasst werden und/oder eine Verjüngung eines Gewindegangs bzw. eine Weitung des korrespondierenden Gewindegangs in endseitiger Längsrichtung implementiert werden.

In einer Variante gemäß dem zweiten Lösungsansatz wird die zwischen Verschlusselement beziehungsweise Aufnahmeteil und dem Anschlussteil eine Relativbewegung mit einer Richtungskomponente in Richtung der Längsachse zulassende Verbindung von an einem der zu verbindenden Bauteile - Verschlusselement beziehungsweise Aufnahmeteil oder Anschlussteil - angeordneten und in Richtung zum anderen Bauteil gerichteten Vorsprüngen, die in am jeweils anderen der miteinander zu verbindenden Bauteile - Anschlussteil oder Verschlusselement beziehungsweise ein dieses tragendes Aufnahmeteil- in randoffene, in Richtung der Längsachse verlaufende Aussparungen einführbar sind, gebildet. Die in Richtung der Längsachse verlaufenden Aussparungen münden jeweils in einen in Umfangsrichtung ausgerichteten Teilbereich, welcher einen Formschluss entgegen der Längsachsrichtung ermöglicht.

Die Vorsprünge können verschiedenartig ausgeführt sein. Denkbar sind hinsichtlich des in die Schlitze eingreifenden Querschnitts kreisrunde, ovale oder mehreckige Querschnittsgeometrien.

Die Vorsprünge und die Aussparungen können in Abhängigkeit der gewählten Materialen für das Anschlussteil oder Verschlusselement beziehungsweise ein dieses tragendes Aufnahmeteil bei der Herstellung dieser mit angeformt beziehungsweise ausgeformt werden oder nachträglich mit diesen verbunden werden.

Gemäß einer besonders vorteilhaften Ausbildung ist die formschlüssige Verbindung derart ausgeführt, zumindest eine Rastposition innerhalb des theoretischen Verbindungsbereiches, jedoch außerhalb des die erste Funktionsstellung definierenden Teilbereiches auszubilden, in welcher ebenfalls ein Formschluss zwischen Verschlusselement und Anschlussteil entgegen der Längsachsrichtung ermöglicht wird. Die Rastposition wird dazu an zumindest einem der Bauteile zwischen den die erste Funktionsstellung bei Verbindung charakterisierenden Teilbereichen der Verbindungsmittel und dem Trennbereich vom anderen Bauteil angeordnet. Im einfachsten Fall werden dazu in den Aussparungen spezielle Rastbereiche bildende Teilaussparungen vorgesehen, die einen die Bewegbarkeit des Verschlusselementes beziehungsweise des Aufnahmeteils in Längsrichtung begrenzenden Formschluss zwischen den Vorsprüngen am Verschlusselement beziehungsweise dem dieses tragenden Bauteil und den Aussparungen am Anschlussteil, ermöglichen.

Unter einem Applikationseinrichtungs-Ansatz kann ein Funktionselement oder ein Teilbereich eines Funktionselementes verstanden werden, welcher ausgeführt und angeordnet ist, geeignet zu sein, mit einer Applikationseinrichtung, wie einer Hohlnadel beziehungsweise Injektionsnadel, einem Schlauch für eine Flügelkanüle o.ä., verbunden zu werden. Dazu kann der Applikationseinrichtungs-Ansatz entsprechende Mittel zur kraft- und/oder formschlüssigen Kopplung mit der Applikationseinrichtung aufweisen, die mit dazu komplementär ausgebildeten Mitteln an der jeweiligen Applikationseinrichtung in Wirkverbindung bringbar sind.

Der Applikationseinrichtungs-Ansatz ist hohlzylindrisch ausgeführt und weist zumindest einen konisch ausgeführten Teilbereich auf. Dieser kann als integrales Bauteil mit einem anderen Funktionselement oder dem Behälter oder als separates Bauteil ohne weitere Funktion ausgeführt sein.

Gemäß einer ersten Grundausbildung für den Applikationseinrichtungs-Ansatz ist dieser von einem am Behälter angeformten Bereich gebildet und der Sicherungsring ist direkt oder über einen, den Applikationseinrichtungs-Ansatz in Umfangsrichtung umschließenden Haltering in Umfangsrichtung und in Richtung der Längsachse am Behälter befestigt.

In einer zweiten Grundausbildung ist der Applikationseinrichtungs-Ansatz von einem separaten Element gebildet oder an einem Funktionselement mit ausgebildet, welches wenigstens mittelbar form- und/oder kraftschlüssig mit dem Behälter verbunden ist.
Das Verschlusssystem weist einen in eine endseitige Öffnung am Behälter einbringbaren, eine Durchgangsöffnung zur Verbindung zwischen Behälterinnenraum und Umgebung aufweisenden Verschlussstopfen mit einer bezogen auf die Längsachse in Umfangsrichtung verlaufenden äußeren Umfangsfläche auf, welche über einen Teilbereich eine Dichtfläche zur Anlage an einem Teilbereich des Innenumfanges des Behälters im Bereich der endseitigen Öffnung bildet. Der Verschlussstopfen ist über eine Überwurfkappe am Behälter kraft- und/oder formschlüssig fixiert und der Applikationseinrichtungs-Ansatz wird von zumindest einem Teilbereich der Überwurfkappe oder einen sich durch die Überwurfkappe erstreckenden Teilbereich am Verschlussstopfen gebildet. In vorteilhafter Ausführung ist das Anschlusselement in diesem Fall integral mit der Überwurfkappe ausgeführt.

In einer alternativen Ausführung ist es auch denkbar, dass der Applikationseinrichtungs-Ansatz vom oder am Verschlussstopfen ausgebildet wird und der Verschlussstopfen ist wenigstens mittelbar kraft- und/oder formschlüssig mit dem Behälter verbunden. Es sind Mittel zur Fixierung des Sicherungsringes in Umfangsrichtung um die Längsachse und in Richtung der Längsachse am Verschlussstopfen und/oder am Behälter vorgesehen.

Das Verschlusssystem ist in besonders vorteilhafter Weise als Baueinheit vorkonfektioniert, falls erforderlich gereinigt und steril verpackt lagerbar und als Baueinheit einem Applikationseinrichtungs-Ansatz zum Verschluss zuordenbar.
Die Reinigung und gegebenenfalls Sterilisation der einzelnen Bauteile kann einzeln oder aber auch für die gesamte vormontierte Baueinheit erfolgen.

Gemäß einer besonders vorteilhaften Weiterbildung beinhaltet die vorkonfektionierbare Baueinheit auch den Applikationseinrichtungs-Ansatz, wobei die Baueinheit nur noch mit dem Behälter verbunden werden muss, insbesondere auf den endseitigen zu verschließenden Bereich aufgeschoben werden muss.

In einer besonders vorteilhaften Weiterbildung wird die Fixierung des Anschlusselementes am Behälter durch Verspannung dessen gegenüber dem Behälter realisiert. Dies kann im einfachsten Fall durch einen zusätzlich vorgesehenen Spannring erfolgen, welcher den Sicherungsring der Verschluss-Sicherungseinrichtung in der zweiten Montagestellung in Umfangsrichtung im Erstreckungsbereich des Anschlusselementes umschließt und diesen gegenüber dem Behälter verspannt. Der erforderliche Druck zum Fixieren, insbesondere Verspannen des Anschlusselementes gegenüber dem Behälter muss somit nicht mehr allein über den behälterseitigen Endbereich des Sicherungsringes aufgebracht werden, so dass die dafür erforderliche Fläche gering gehalten werden kann, was zu einer Verringerung der erforderlichen Erstreckung des Sicherungsringes und der Anschlussfläche am Anschlusselement führt, die sich in einer Verringerung der Baugröße des Verschlusssystems gegenüber der erfindungsgemäßen Ausführung ohne Spannring niederschlägt.

Eine besonders vorteilhafte Verwendung eines Verschlusssystems gemäß einem der Ansprüche 1 bis 20 besteht in der Verwendung zum Verschließen eines zumindest ein pharmazeutisches Präparat aufnehmenden Behälters, insbesondere Doppelkammer-Behälters, um ein steriles Verschließen noch im Lyophilisator zu gewährleisten und ferner beim Lösen des Verschlusselementes einen einfachen und sicheren Spritzschutz sowie eine Entlüftung zu ermöglichen.

Der erfindungsgemäße Behälter zur Aufnahme pharmazeutischer Präparate weist einen an diesem ausgebildeten oder befestigbaren Applikationseinrichtungs-Ansatz mit einem Verschlusssystem gemäß einem der Ansprüche 1 bis 20 auf. Dieser ist in besonders vorteilhafter Weise als Doppelkammer-Behälter mit einem Mundstück zur Ausbildung der Öffnung und zum Anschluss des Applikationseinrichtungs-Ansatzes ausgebildet, wobei das Verschlusssystem mit Positionierung der Verschluss-Sicherungseinheit gegenüber dem Anschlusselement bereits vor Einbringung in den Lyophilisator dem Behälter zugeordnet werden kann und im Lyophilisator die Lyophilisierung und nach dieser ein steriles Verschließen noch innerhalb des Lyophilisators erfolgen kann.

Zur Eignung des Behälters und des Verschlusssystems zur Lyophilisation ist dieser als Doppelkammer-Behälter mit einem Mundstück zur Ausbildung der Öffnung und zum Anschluss des Applikationseinrichtungs-Ansatzes ausgebildet, wobei das Mundstück zumindest zwei komplementär zu den Mitteln am Anschlusselement ausgebildete Mittel zur Realisierung eines Kraft- und/oder Formschlusses umfasst, die in Längsrichtung zueinander versetzt angeordnet sind, um in der ersten Montagestellung des Verschlusssystems das Anschlusselement in einer ersten Lage gegenüber dem Behälter zu halten und in der zweiten Montagestellung des Verschlusssystems das Anschlusselement in einer zweiten Lage gegenüber dem Behälter zu fixieren.

In besonders vorteilhafter Weise findet der Behälter mit einem derartigen Verschlusssystem als Spritze für medizinische Zwecke Verwendung. Dabei wird das bei Entlüftung des Behälters gemäß dem Stand der Technik und während der Rekonstitution mögliche Herausspritzen von Lösung sicher unterbunden.

Ein erfindungsgemäßes Verfahren zur Montage eines Behälters mit einem derartigen Verschlusssystem, insbesondere derartigen Spritze ist durch nachfolgende Verfahrensschritte charakterisiert:
- Vormontage des Verschlusssystems aus zumindest einem Verschlusselement und Verschluss-Sicherungseinheit zu einer Baueinheit
- Sterilisation und optional falls erforderlich Reinigung des Verschlusssystems
- Zuordnung zu einem Behälter und Verschluss eines endseitigen Bereiches eines pharmazeutische Produkte aufnehmenden Behälters.

In einer besonders vorteilhaften Weiterentwicklung wird auch ein Applikationseinrichtungs-Ansatz mit in der Baueinheit vorkonfektioniert.

Beispielsweise kann das komplette Verschlusssystem zuerst in eine Gefriertrocknungsposition auf den Behälter, insbesondere einen Doppelkammer-Behälter einer Spritze aufgesetzt werden und nach dem Gefriertrocknungsprozeß die Spritze durch Zusammenfahren der Gefriertrocknungsplatten (Krafteinwirkung auf Verschlusssystem) komplett verschlossen werden.
Das Verschließen des Behälters innerhalb eines Lyophilisators erfolgt durch folgende Schritte:
- Zuordnung des Verschlusssystems zum Behälter mit der Verschluss-Sicherungseinheit in einer ersten Montagestellung gegenüber dem Anschlusselement vor der Lyophilisierung (kann auch außerhalb des Lyophilisators erfolgen) und
- Verschiebung der Verschluss-Sicherungseinheit in eine zweite Montagestellung gegenüber dem Anschlusselement unter Fixierung dieser am Behälter nach der Lyophilisierung.

In besonders vorteilhafter Ausführung wird in der ersten Montagestellung das Anschlusselement am die Öffnung aufweisenden Endbereich des Behälters diesen in Umfangsrichtung umschließend in einer ersten Stellung angeordnet und die Verschluss-Sicherungseinheit und das mit dieser gekoppelte Verschlusselement in Längsrichtung betrachtet gegenüber dem Anschlusselement in einer Lage frei von einer Abdichtung des Applikationseinrichtungs-Ansatzes positioniert. Das Verschließen des Behälters während oder nach einer Lyophilisation erfolgt durch Verbringen des Verschlusssystems in eine zweite Montagestellung unter Relativbewegung zumindest einzelner Komponenten des Verschlusssystems zueinander und/oder gegenüber dem die Öffnung beinhaltenden Endbereich, insbesondere Verschiebung der Verschluss-Sicherungseinheit und des Verschlusselementes relativ zum Anschlusselement in Längsrichtung unter dichtendem Verschließen des Applikationseinrichtungs-Ansatzes und Verschiebung des Anschlusselementes am Endbereich des Behälters unter Fixierung des Verschlusssystems am Behälter nach der Lyophilisierung in eine zweite Stellung.

Die Materialen für das Verschlusselement und den Verschlussstopfen sind nicht besonders beschränkt. Vorzugsweise wird ein elastisches, flexibles Material verwendet. Beispiele hierfür sind Gummi, Kautschuk, wie natürlicher oder synthetischer Kautschuk, Kunststoffe, wie Elastomere, Thermoplaste, thermoplastische Elastomere. Vorzugsweise finden dampf- und/oder ethylenoxiddurchlässige Gummiformulierungen Anwendung, wie beispielsweise Brombutyle, Polyisoprene, Mischungen der vorgenannten.
Das Material kann auch mit einem Überzug versehen sein, wodurch zum Beispiel eine Barriere für Leachables geschaffen werden kann.

Der Behälter ist als Einkammer- oder Mehrkammer - Behälter, insbesondere Doppelkammer-Behälter, welcher einen zylindrischen Körper umfasst, ausgeführt. Bei diesem handelt es sich um einen im wesentlichen länglichen und hohlen Körper mit zwei offenen Enden, der vorzugsweise einstückig ist, d.h. in einem Stück hergestellt wurde, und bei Ausführung als Doppelkammer-Behälter durch Trennstopfen (Mittel- und Endstopfen) in Kammern unterteilt wird. Das Material aus dem der zylindrische Körper besteht oder das er enthält, ist nicht besonders beschränkt. Der Behälter kann aus Kunststoffen oder Glas gefertigt werden. Glas wird aufgrund seiner Transparenz, und Kompatibilität mit vielen pharmazeutischen Formulierungen verbreitet verwendet. Der zylindrische Körper besteht daher vorzugsweise aus Glas oder enthält dieses, da hierdurch die geringste Beeinträchtigung der enthaltenen Komponenten resultiert und der Körper vorzugsweise transparent ist. Für besondere Anforderungen sind aber auch andere Materialien geeignet, wie spezielle Kunststoffe oder dergleichen. Hierbei spielt insbesondere die pharmazeutische Unbedenklichkeit eine Rolle, da eine möglichst geringe Wechselwirkung mit dem enthaltenen Medium anzustreben ist.
Vorzugsweise weist der zylindrische Körper über die gesamte Länge denselben Durchmesser auf.

In der vorliegenden Erfindung soll der Begriff "Form" oder "Kontur" die äußere Form oder Geometrie bezeichnen. Der Begriff "Dimension" soll die Abmessungen, d.h. Größenverhältnisse, bezeichnen.

Die erfindungsgemäße Lösung wird nachfolgend anhand von Figuren erläutert. Darin ist im Einzelnen Folgendes dargestellt:

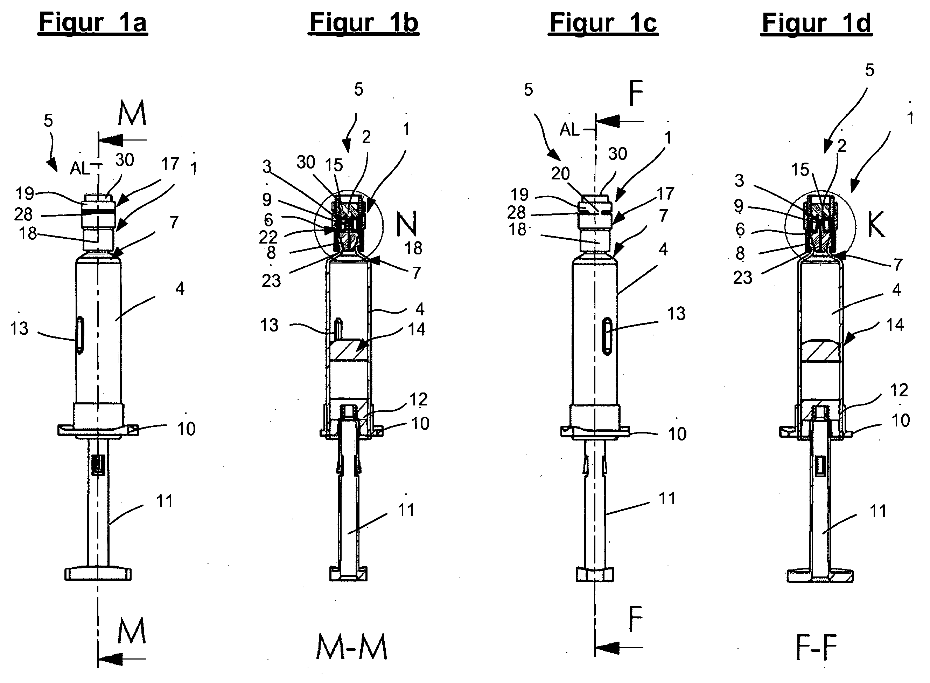

- Figuren 1a bis 1d: verdeutlichen in schematisiert vereinfachter Darstellung den Grundaufbau und die Grundfunktion eines erfindungsgemäßen Verschlusssystems gemäß einer ersten Variante einer ersten Ausführung für einen eine Durchgangsöffnung aufweisenden Applikationseinrichtungs-Ansatz in Form eines separaten Funktionselementes zur Kopplung mit einem Behälter am Beispiel einer Spritze;

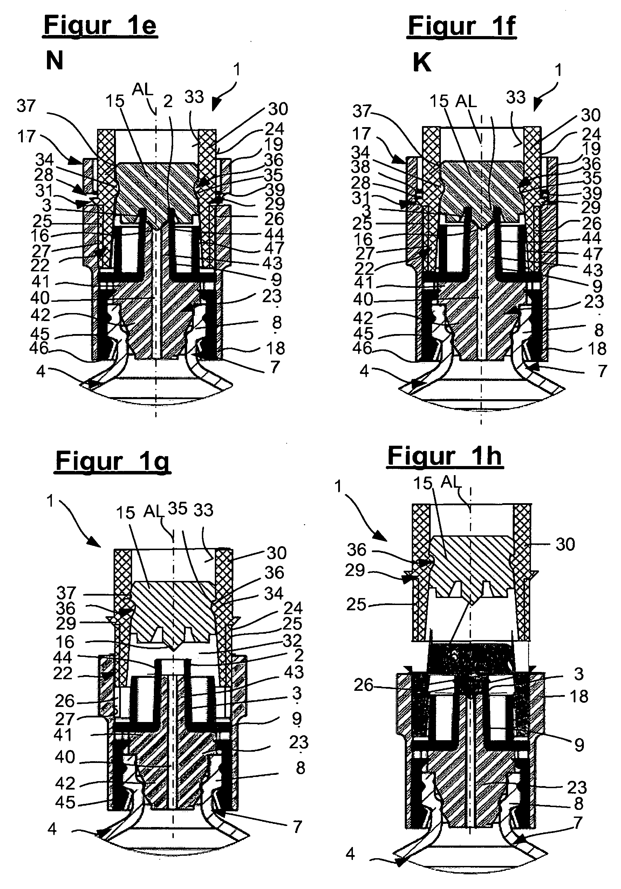

- Figur 1e: zeigt ein Detail N gemäß einer Ansicht M-M aus Figur 1b;
- Figur 1 f: zeigt ein Detail K gemäß einer Ansicht F-F aus Figur 1d;
- Figur 1g: verdeutlicht in einer Ansicht gemäß Figur 1e das Verschlusssystem in der zweiten Funktionsstellung des Verschlusselementes;
- Figur 1h: verdeutlicht in einer Ansicht gemäß Figur 1f die Komponenten des Verschlusssystems nach vollständigem Lösen des Verschlusselementes;
- Figuren 2a bis 2d: verdeutlichen in schematisiert vereinfachter Darstellung den Grundaufbau und die Grundfunktion eines erfindungsgemäßen Verschlusssystems gemäß einer zweiten Variante einer ersten Ausführung für einen eine Durchgangsöffnung aufweisenden Applikationseinrichtungs-Ansatz in Form eines separaten Funktionselementes zur Kopplung mit einem Behälter am Beispiel einer Spritze;
- Figur 2e: zeigt ein Detail L gemäß einer Ansicht J-J aus Figur 2b;
- Figur 2f: zeigt ein Detail I gemäß einer Ansicht H-H aus Figur 2d;
- Figur 3a: zeigt das Verschlusssystem in einer Ansicht gemäß Figur 1c;
- Figur 3b: zeigt einen Schnitt K-K aus Figur 3a;
- Figur 4: zeigt eine Ansicht von oben auf das Aufnahmeteil;
- Figur 5a: zeigt anhand einer Ansicht gemäß Figur 1e beispielhaft eine weitere Ausbildung einer eine Relativbewegung zulassenden Verbindung als formschlüssige Verbindung zwischen Verschlusselement beziehungsweise dem dieses tragenden Aufnahmeteil und Anschlussteil;
- Figur 5b: zeigt die Verbindungsmittel der eine Relativbewegung zulassenden Verbindung am Sicherungsring;
- Figur 5c: zeigt die Verbindungsmittel der eine Relativbewegung zulassenden Verbindung am Aufnahmeteil;
- Figuren 5d1 und 5d2: zeigen mögliche Ausgestaltungen der Ausnehmung mit Rastposition;
- Figur 6: verdeutlicht ein erfindungsgemäßes Verschlusssystems gemäß einer zweiten Ausführung mit direkter Verbindung zwischen Verschlusselement und Anschlussteil, insbesondere Sicherungsring in einem Axialschnitt;
- Figur 7: verdeutlicht ein erfindungsgemäßes Verschlusssystem gemäß einer zweiten Ausführung mit direkter Verbindung zwischen Verschlusselement und Anschlussteil, hier beispielhaft in Form einer Überwurfkappe in einem Axialschnitt;
- Figuren 8a und 8b: zeigen anhand der Ausführung der Überwurfkappe und des Verschlusselementes eine alternative Ausbildung des Verschlusssystems gemäß Figur 7;
- Figuren 9a und 9b: zeigen anhand einer Schnittdarstellung und einer Ansicht von Rechts eine weitere Ausführung eines erfindungsgemäßen Verschlusssystems;
- Figur 10: zeigt beispielhaft anhand einer Ausführung eines Verschlusssystems gemäß der Figuren 2a bis 2f die Positionierung der einzelnen Komponenten zueinander und gegenüber dem Behälter vor dem Verschließen im Lyophilisator;
- Figur 11a: zeigt beispielhaft eine besonders vorteilhafte Weiterentwicklung eines Verschlusssystems an einem Behälter mit einem Spannring;
- Figur 11b: zeigt eine Ausführung gemäß Figur 11a in einer ersten Montagestellung;
- Figur 12a: verdeutlicht in schematisiert vereinfachter Darstellung den Ablauf eines Verfahrens zur Herstellung einer Spritze;
- Figur 12b: verdeutlicht in schematisiert vereinfachter Darstellung den Ablauf eines Verfahrens beim Öffnen des Verschlusssystems.
- Figur 13a: zeigt eine alternative Ausbildung des Applikationseinrichtungs-Ansatzes am Verschlussstopfen;
- Figur 13b: zeigt eine alternative Ausbildung des Applikationseinrichtungs-Ansatzes am Behälter.

In den Figuren haben gleiche Teile die gleichen Bezugszeichen.
Die Figuren 1a bis 1f zeigen eine Ausführung eines erfindungsgemäßen Verschlusssystems 1 gemäß einer ersten Variante einer ersten Ausführung für einen eine Durchgangsöffnung 2 aufweisenden Applikationseinrichtungs-Ansatz 3 in Form eines separaten Funktionselementes zur Kopplung mit einem Behälter 4, der in einer besonders vorteilhaften Anwendung als Doppelkammer-Behälter für den Einsatz als Spritze ausgeführt ist. Die Figuren 1a und 1c zeigen dabei eine Spritze nach der Lyophilisierung im verschlossenen Zustand des Behälters 4 in zwei verschiedenen Ansichten. Die Figur 1b zeigt einen Schnitt M-M gemäß Figur 1a, die Figur 1d einen Schnitt F-F gemäß Figur 1c. Das Verschlusssystem 1 ist in den Figuren 1a bis 1f in seiner ersten Funktionsstellung wiedergegeben, d.h. der Verschlussstellung, in welcher der Behälter 4 dichtend verschlossen ist.

Das separate, den Applikationseinrichtungs-Ansatz 3 bildende Funktionselement ist als Überwurfkappe 9 ausgebildet und dient neben der Ausbildung als Applikationseinrichtungs-Ansatz 3 der Lagefixierung eines die Öffnung eines Behälters 4 verschließenden Verschlussstopfens 23 in Richtung einer Längsachse AL des Verschlusssystems 1, die im verschlossenen Zustand des Behälters 4 mit der Längsachse des Behälters 4 zusammenfällt. Die Überwurfkappe 9 ist in dieser vorteilhaften Ausbildung ferner integral mit einem mit dem Behälter 4 verbundenen Anschlusselement 57 ausgebildet, d.h. bildet dieses in einem Teilbereich mit aus. Die Verbindung des Anschlussteils 57 mit dem Behälter 4 kann kraft- und/oder formschlüssig erfolgen.

Zur Charakterisierung der Richtungsangaben wird auf eine theoretische Längsachse AL Bezug genommen, die in Längsrichtung ausgerichtet ist und der Mittenachse der Komponenten in Einbaulage entspricht. In Richtung der Längsachse AL bedeutet dabei in Längsrichtung und in Umfangsrichtung bedeutet um diese. Die Figuren 1e und 1f zeigen jeweils das Verschlusssystem 1 mit dem Applikationseinrichtungs-Ansatz 3 im Schnitt gemäß der Details N und K aus den Figuren 1b und 1d.

Der Behälter 4 ist zylindrisch ausgebildet und beschreibt einen um eine Längsachse AL angeordneten Hohlraum, wobei der Querschnitt des Hohlraumes über zumindest einen erheblichen Teilbereich der Erstreckung des Behälters 1 in Richtung der Längsachse AL vorzugsweise kreisförmig oder oval ausgeführt ist. Der Behälter 4 dient der Aufnahme und Lagerung eines oder mehrerer pharmazeutischer Produkte, die als pharmazeutisches Präparat in Reinform oder Mischung vorliegen können und die über eine Öffnung 6 in einem Endbereich 7 des Behälters 4 aus diesem über den Applikationseinrichtungs-Ansatz 3 ausbringbar sind. Der Applikationseinrichtungs-Ansatz 3 ist am Behälter 4 wenigstens mittelbar, vorzugsweise direkt fixiert. Die Verbindung erfolgt form- und/oder kraftschlüssig. Wenigstens mittelbar bedeutet direkt oder über weitere zwischengeordnete Elemente und Komponenten.

Der Behälter 4 weist in einem ersten Endbereich 7 ein Mundstück 8 zur Ausbildung der Öffnung 6 und zum Anschluss des Applikationseinrichtungs-Ansatzes 3 auf. Das Verschlusssystem 1 zum Verschließen des Applikationseinrichtungs-Ansatzes 3 ist derart angeordnet und ausgebildet, geeignet zu sein, im Verschlusszustand in einem ersten Funktionszustand (geschlossen), welcher in den Figuren 1a bis 1f wiedergegeben ist, die mit dem Innenraum des Behälters 4 verbundene Durchgangsöffnung 2 des Applikationseinrichtungs-Ansatzes 3 und damit den Behälter 4 im Endbereich 7 vollständig druckdicht, mediendicht und mikrobiell dicht zu verschließen und in einem weiteren Funktionszustand (geöffnet) die Entnahme oder Abgabe des Präparates aus dem Behälter 4 zu ermöglichen. Bei der Ausbildung der Anordnung aus Behälter 4 und Applikationseinrichtungs-Ansatz 3 als Spritze 5 weist diese eine Fingerauflage 10 sowie wenigstens einen durch wenigstens einen Teilbereich des vom Behälter 4 umschlossenen Innenraumes in Richtung der Längsachse AL geführten Kolben 11 betätigbaren Stopfen 12 auf. Dieser umfasst einen Bypass 13 sowie einen weiteren Stopfen 14, der vor dem Gebrauch des Behälters 4 zwei Präparate und/oder Lösungen, beispielsweise in lyophilisierter Form eingebrachtes bzw. im Behältnis lyophilisiertes pharmazeutisches Präparat von einem Lösungsmittel getrennt hält, das unmittelbar vor Anwendung der Spritze 5 über den Bypass 13 in die dem Mundstück 8 vorgeordnete Kammer überführt wird. Zur Gewährleistung der vorgenannten Funktionen - druckdichter, mediendichter und mikrobiell dichter Verschluss der Öffnung 2 des Applikationseinrichtungs-Ansatzes 3, Entnahme eines pharmazeutischen Präparates über die Durchgangsöffnung 2 und Ankopplung an eine Applikationseinrichtung zur Applikation des pharmazeutischen Präparates - ist das Verschlusssystem 1 vorzugsweise mehrteilig ausgeführt. Vorzugsweise erfolgt die Ausführung der einzelnen Komponenten zylindrisch um die Längsachse AL.

Das Verschlusssystem 1 umfasst zumindest ein der Durchgangsöffnung 2 des Applikationseinrichtungs-Ansatzes 3 zugeordnetes und wenigstens eine Dichtfläche 16 zur Abdichtung dieser aufweisendes Verschlusselement 15, welches zum Anschluss der Applikationseinrichtung an den Applikationseinrichtungs-Ansatz 3 entfernbar ist. Das Verschlusselement 15 ist gemäß einer ersten Variante der ersten Ausführung in einem Aufnahmeteil 30 formschlüssig gehalten. Das Verschlusselement wird auch als Tip-Cap bezeichnet.

Zur Gewährleistung der Reinheit und Sterilität bis zum tatsächlichen Gebrauch ist eine Verschluss-Sicherungseinheit 17 vorgesehen. Die Verschluss-Sicherungseinheit 17 umschließt das Verschlusselement 15 und zumindest den freien, d.h. vom Behälter 4 weggerichteten Endbereich des Applikationseinrichtungs-Ansatzes 3 über einen Teilbereich der Erstreckung in Richtung der Längsachse AL in Umfangsrichtung und umfasst einen den Applikationseinrichtungs-Ansatz 3 in Umfangsrichtung über einen Teilbereich seiner Erstreckung in Längsrichtung umschließenden und an diesem und/oder dem Behälter 4 wenigstens mittelbar fixierten Sicherungsring 18. Zusätzlich ist in einer besonders vorteilhaften Ausgestaltung zur Gewährleistung des Orginalitätsverschlusses ein mit dem Sicherungsring 18 lösbar über Verbindungsmittel 20 unter Zerstörung dieser verbundenes Sicherungsteil 19 vorgesehen, welches ringförmig um das Verschlusselement 15 erstreckend, vorzugsweise unter Ausbildung eines Abstandes in radialer Richtung bezogen auf die Längsachse AL verlaufend ausgeführt ist. Zwischen Verschlusselement 15 und dem Sicherungsteil 19 ist zumindest mittelbar ein in Richtung der Längsachse wirksamer Formschluss 31 vorgesehen. Im dargestellten Fall wird der Formschluss 31 über das Verschlusselement 15 aufnehmende Aufnahmeteil 30 realisiert.

Die Verschluss-Sicherungseinheit 17 ist kraft- und/oder formschlüssig über das Anschlusselement 57 am Behälter 2 befestigt. Das Anschlusselement 57 ist hülsenartig ausgeführt und umgreift den Öffnungsbereich des Behälters 4. Die Verbindung zwischen Anschlusselement und Behälter 4 erfolgt kraft- und/oder formschlüssig. Vorzugsweise ist das Anschlusselement 57 in seinem zum Behälter weisenden Endbereich mit stegartigen Rastelementen ausgeführt, die beim Umgreifen des Anschlusselementes 57 durch den Sicherungsring 18 gegenüber dem Behälter 4 fixiert werden, insbesondere in dafür vorgesehene Aussparungen und Hinterschneidungen eingreifen.

Um bei der Rekonstitution beziehungsweise Mischung der im Doppelkammer-Behälter enthaltenen Produkte beziehungsweise Präparate eine Entlüftung zu ermöglichen, wobei gleichzeitig ein Spritzschutz gewährleistet werden soll, ist das Verschlusselement 15 wenigstens mittelbar mit einem Anschlussteil, als welches hier gemäß einer ersten Variante der Sicherungsring 18 fungiert, lösbar kraft- und/oder formschlüssig verbunden. Die Verbindung ist mit 22 bezeichnet und wird zwischen dem das Verschlusselement 15 aufnehmenden Aufnahmeteil 30 und dem Sicherungsring 18 realisiert. Die Verbindung 22 ist derart angeordnet und ausgeführt, geeignet zu sein, nach Abtrennung des Sicherungsteils 19 vom Sicherungsring 18 unter Aufhebung des in Richtung der Längsachse AL wirksamen Formschlusses 31 zwischen Verschlusselement 15 beziehungsweise dem dieses tragenden Funktionselement, hier dem Aufnahmeteil 30 und dem Sicherungsteil 19, eine Relativbewegung zwischen Verschlusselement 15 und Applikationseinrichtungs-Ansatz 3 mit einer Richtungskomponente in Richtung der Längsachse AL aus der ersten Funktionsstellung des Verschlusselementes 15 (Verschluss-Stellung) in zumindest eine weitere, in der Figur 1g für die Ansicht gemäß Figur 1e wiedergegebene Funktionsstellung (Spritzschutz- und/oder Entlüftungsstellung) unter zumindest teilweiser Aufrechterhaltung dieser Verbindung 22 zu ermöglichen, wobei in der zumindest einen weiteren Funktionsstellung die druckdichte, mikrobiell dichte und mediendichte Abdichtung der Durchgangsöffnung 2 am Applikationseinrichtungs-Ansatz 3 durch das Verschlusselement 15 unter Bildung eines vom Verschlusselement 15 und Anschlussteil, hier auch dem das Verschlusselement 15 tragenden Aufnahmeteil 30 gebildeten Zwischenraumes 32 aufgehoben ist. Dieser Zwischenraum 32 erlaubt bereits eine Druckfortpflanzung in diesen. Unter Druck aus dem Behälter 4 austretendes Medium wird in diesem Zwischenraum 32 aufgefangen und nicht unkontrolliert in die Umgebung verspritzt.

In einer Ausführung könnte bereits eine Relativbewegung innerhalb der Verbindung 22 ausreichen, beispielsweise durch minimales Herausdrehen des Verschlusselementes 15, so dass spritzende Lösung gar nicht in den Zwischenraum 32 gelangt, sondern am kegelförmigen Teil des Verschlusselements (Dichtfläche 16) "kondensiert" und in den von einer Durchgangsöffnung 40 gebildeten Kanal des Verschlussstopfens 42 zurückfließt.

In einer besonders vorteilhaften Weiterbildung sind Entlüftungsmittel 21 vorgesehen, die entweder von den die Verbindung 22 realisierenden Verbindungsmitteln beziehungsweise Verbindungselementen am Verschlusselement 15 beziehungsweise dem dieses tragenden Aufnahmeteil 30 und einem Anschlussteil gebildet werden oder aber als separate Kanäle in die Verbindung 22 eingearbeitet sind.

Das Aufnahmeteil 30 ist als sich in Richtung der Längsachse AL erstreckendes hohlzylindrisches Element ausgeführt, welches in einem Teilbereich der Erstreckung seines Innenumfanges 33 in Längsrichtung Verbindungsmittel 34 umfasst, die mit dazu komplementär ausgeführten Verbindungsmitteln an einem Bereich des Außenumfanges 37 des Verschlusselementes 15 in Wirkverbindung unter Ausbildung einer formschlüssigen Verbindung 36 bringbar sind. Das Verschlusselement 15 ist hinsichtlich seines Außenumfanges 37 an den Innenumfang 33 des Aufnahmeteils 30 angepasst ausgeführt. Das als holzylindrisches Element ausgeführte Aufnahmeteil 30 kann bezogen auf die Längsachse AL in Längsrichtung mit unveränderter geometrischer Form und/oder Abmessungen oder aber mit Bereichen unterschiedlicher geometrischer Form und/oder Abmessungen in radialer Richtung und/oder Längsrichtung ausgeführt sein. Im dargestellten Fall wird die Aufnahme- und Tragfunktion mittels Formschluss durch eine Rastverbindung erzeugt, indem am Innenumfang 33 angeordnete Vorsprünge in dazu komplementär ausgebildete Ausnehmungen am Verschlusselement 15 eingreifen. Die Vorsprünge und Ausnehmungen sind in besonders vorteilhafter Ausbildung vollständig in Umfangsrichtung umlaufend an den jeweiligen Komponenten ausgebildet, d.h. als umlaufender Ring am Innenumfang 33 und Nut oder Rille am Außenumfang 37 des Verschlusselementes 15. Denkbar ist auch eine Ausführung mit sich über einen Teilbereich des Außen- beziehungsweise Innenumfanges erstreckenden Aussparungen und Vorsprüngen, die jeweils in gleichem Abstand zu einander oder aber mit unterschiedlichem Abstand zueinander angeordnet sein können. Ebenfalls denkbar ist die hier nicht dargestellte alternative Ausgestaltung mit am Außenumfang 37 des Verschlusselementes 15 angeordneten, in radialer Richtung ausgerichteten Vorsprüngen und dazu komplementären Aussparungen am Innenumfang 33 des Aufnahmeteils 30. Die Anordnung des Verschlusselementes 15 im Aufnahmeteil 30 erfolgt derart, dass der die Dichtfläche 16 tragende Bereich in Einbaulage zum Applikationseinrichtungs-Ansatz 3 gerichtet ist. Die Dichtfläche 16 wird von einem in Richtung der Längsachse AL an der zum Applikationseinrichtungs-Ansatz 3 gerichteten Seite hervorstehenden Vorsprunges, insbesondere Dichtlippe mit einem konisch ausgeführten Endbereich gebildet.

Die Verbindung 22 ist im in den Figuren 1a bis 1f dargestellten Fall als Schraubverbindung mit hier im Einzelnen nicht dargestellten integrierten Entlüftungskanal ausgeführt. Zur Realisierung der Verbindung 22 umfasst das Verschlusselement 15 tragende Aufnahmeteil 30 wenigstens ein an einem Teilbereich seiner äußeren Umfangsfläche 31 mit einer Richtungskomponente in Längsrichtung und über einen Teilbereich der Erstreckung der Umfangsfläche 24 in Längsrichtung angeordnetes Außengewinde 25, welches mit einem Innengewinde 26 an einem, sich in Richtung der Längsachse AL erstreckenden und einen Innenumfang bildenden Teilbereich 27, der in Umfangsrichtung um die Längsachse AL verlaufend an einem Anschlussteil, hier dem Sicherungsring 18 ausgeführt ist, eingreift. Der Entlüftungskanal wird beispielsweise über eine Unterbrechung des Gewindes am Außengewinde 25 und/oder Innengewinde 26 realisiert, wobei vorzugsweise ein sich in Richtung der Längsachse AL erstreckender Kanal eingearbeitet ist. Die Verbindungsmittel am Aufnahmeteil 30, hier das Außengewinde 25, sind in Einbaulage hier im zum Applikationseinrichtungs-Ansatz 3 gerichteten Endbereich des Aufnahmeteils 30 angeordnet.

Denkbar wäre auch eine hier nicht dargestellte Ausführung, bei der das Element 30 so gestaltet ist, dass dieses ein Innengewinde trägt und der Sicherungsring 18 ein Außengewinde.

Die Verschlusssicherungsfunktion zur Gewährleistung eines einmaligen Gebrauchs unter sterilen Bedingungen unter Vermeidung eines unerwünschten Auf- und Wiederverschließens des Mundstückes 8 (Orginalitätsverschlussfunktion) wird über die Abtrennbarkeit des Sicherungsteils 19 vom Sicherungsring 18 unter irreversibler Zerstörung der Verbindung und den in Längsrichtung zumindest in Längsrichtung vom Behälter 4 beziehungsweise dem Applikationseinrichtungs-Ansatz 3 weggerichtet wirkenden Formschluss 31 zwischen Verschlusselement 15 beziehungsweise bei Integration im Aufnahmeteil 30 dem Aufnahmeteil 30 realisiert, welcher bei Abtrennung des Sicherungsteils 19 aufgehoben wird. Der in Längsrichtung wirksame Formschluss 31 ist dazu im Verbindungsbereich 28 zwischen Sicherungsring 18 und Sicherungsteil 19 angeordnet und wird zwischen Sicherungsteil 19 und Aufnahmeteil 30 realisiert. Das Verschlusselement 15 tragende Aufnahmeteil 30 weist einen sich an der Umfangsfläche 24 am Außenumfang des Aufnahmeteils 30 in Einbaulage außerhalb des das Außengewinde 25 tragenden Teilbereichs vorzugsweise vollständig in Umfangsrichtung um die Längsachse AL umlaufenden ringförmigen elastischen Sicherungsvorsprung 29 auf, der im Verschlusszustand des Applikationseinrichtungs-Ansatzes 3 im Verbindungsbereich 28 in den außerhalb der Verbindungsmittel 20 gebildeten ringsegmentförmigen Raum und eine Ausnehmung 38 im Anordnungsbereich der Verbindungsmittel 20, welche eine in Löserichtung des Verschlusselementes 15 gegen diese wirkende Hinterschneidung für den Sicherungsvorsprung 29 bilden, eingreift. Die durch die Ausnehmung 38 gebildete Hinterschneidung ist in der Detailansicht K in Figur 1f wiedergegeben. Figur 1e zeigt die für den entgegen der Löserichtung des Verschlusselementes 15 in Längsrichtung wirkenden Formschluss verantwortliche, zum Sicherungsring 18 gerichtete Fläche 39 des Sicherungsteils -9. Der ringförmige Sicherungsvorsprung 29 ist elastisch ausgeführt und hinsichtlich einer geometrischen Ausführung und Dimensionierung derart gewählt, dass beim Eindrehen des Aufnahmeteils 30 in den Sicherungsring 18 der Sicherungsvorsprung 29 sich entgegen der Bewegungsrichtung entlang der Längsachse AL an den Außenumfang des Aufnahmeteils 30 verformend anlegt und bei Erreichen der Verschluss-Stellung des Verschlusselementes 15 gegenüber dem Applikationseinrichtungs-Ansatz 3 der Sicherungsvorsprung 29 in den im Verbindungsbereich 28 zwischen Sicherungsteil 19 und Sicherungsring 18 gebildeten Ringraum eingreift, in diesen ausklappt und an der vom Sicherungsteil 19 in Längsrichtung zum Sicherungsring 18 gerichteten Fläche 39 zum Anliegen gelangt oder bei Zulassen einer geringfügigen Relativbewegung beabstandet zu dieser ausgerichtet ist. Um nunmehr das Aufnahmeteil 30 mit dem Verschlusselement 15 wieder herauszudrehen bedarf es einer Kraft, die eine Zerstörung der Verbindungsmittel 20, zwischen Sicherungsteil 19 und Sicherungsring 18 bewirkt.

Die Elastizität des ringförmigen Sicherungsvorsprunges 29 kann als Funktion des Materials, der Geometrie und/oder Dimensionierung des Sicherungsvorsprunges 29 definiert werden. Vorzugsweise ist im dargestellten Fall der Sicherungsvorsprung 29 im Querschnitt betrachtet mit unterschiedlichen Abmessungen in Längsrichtung in Bewegungsrichtung zum Applikationseinrichtungs-Ansatz 3, d.h. im dargestellten Fall in Eindrehrichtung gestaltet. Die größte Erstreckung in radialer Richtung bezogen auf die Längsachse AL in radialer Richtung weist der Sicherungsvorsprung 29 in Erstreckungsrichtung des Aufnahmeteils 30 betrachtet im von den Verbindungsmitteln zur Realisierung der Verbindung 22 weggerichteten Bereich auf. Die minimalste Erstreckung bezogen auf die Längsachse AL in radialer Richtung weist der Sicherungsvorsprung 29 auf seiner zur Verbindung 22 gerichteten Seite auf. In idealisierter Form ist der umlaufende Sicherungsvorsprung 29 durch einen in Richtung des die Verbindungsmittel, insbesondere Außengewinde 25 tragenden Teilbereiches am diesem konisch verjüngenden Verlauf charakterisiert.

Der Sicherungsvorsprung 29 kann - hier nicht dargestellt - auch von Ringsegmenten gebildet werden. Denkbar sind auch Ausführungen mit einzelnen elastischen, in Umfangsrichtung um das Aufnahmeteil 30 beabstandet angeordneten Vorsprüngen.

Der Applikationseinrichtungs-Ansatz 3 wird bei dieser Ausführung in vorteilhafter Weise von einer Überwurfkappe 9 gebildet, die als separates Bauteil am Mundstück 8 ansetzbar und befestigbar ist. Das dargestellte Verschlusssystem 1 umfasst dazu einen Verschlussstopfen 23, der in die Öffnung 6 des Mundstückes 8 im Endbereich 7 des Behälters 4 einbringbar ist und derart ausgeführt ist, geeignet zu sein mit einem Teilbereich seines Außenumfanges dicht an einem Teilbereich des Innenumfanges des Mundstücks 8 anzuliegen. Der Verschlussstopfen 23 ist ferner durch zumindest eine, einen Verbindungskanal bildende Durchgangsöffnung 40 charakterisiert, die sich durch den Verschlussstopfen 23 in Einbaulage von einer in Längsrichtung betrachtet zum Behälter 4 weisenden Fläche zu seiner vom Behälter 4 weggerichteten Fläche erstreckt. Der Verschlussstopfen 23 ist im Verbindungsbereich 42 mit dem Behälter 4 hinsichtlich seiner Form und Dimension an die der Öffnung 6 im Verbindungsbereich am Behälter 4 angepasst, hier vorzugsweise als rotationssymmetrisches Element mit kreisförmigen Querschnitt. Vorzugsweise ist die Außenkontur des Verschlussstopfens 23 in Umfangsrichtung betrachtet derart gewählt, dass dieser entweder mit Übermaß in die Öffnung 6 einpressbar ist und/oder über eine druck- und mediendichte formschlüssige Verbindung im Mundstück 8 fixiert ist. Im dargestellten Fall weist der Verschlussstopfen 23 am Außenumfang in Umfangsrichtung wenigstens über einen Teilbereich umlaufende Vorsprünge auf, die hinter entsprechenden Vorsprüngen am Innenumfang des Mundstückes 8 zum Anliegen gelangen. Der Verschlussstopfen 23 weist ferner einen Bund 41 auf, der eine erste behälterseitige Anlagefläche zur Anlage am Behälter 4, insbesondere am Mundstück 8 und eine zweite vom Behälter 4 weggerichtete und damit applikationseinrichtungsseitige Anlagefläche bildet, an welcher der Applikationseinrichtungs-Ansatz 3, hier die diesen bildende Überwurfkappe 9 anliegt.

Der Verschlussstopfen 23 umfasst neben dem Verbindungsbereich 42 mit dem Behälter 4 einen sich von diesem in Richtung der Längsachse AL weggerichteten Endbereich 43, der konisch in Anpassung an die Innenkontur der Durchgangsöffnung 2 des Applikationseinrichtungs-Ansatzes 3 angepasst ist, sich bis in den vom Applikationseinrichtungs- Ansatz 3 umschlossenen Raum im Ansatzbereich erstreckt und durch welchen der Verbindungskanal 40 bis in den Ansatzbereich geführt wird, um eine dichte Verbindung zwischen Behälterinnenraum und Applikationseinrichtungs-Ansatz 3 zu gewährleisten. Der Verschlussstopfen 23 kann auch mit einem Überzug versehen sein, wodurch zum Beispiel eine Barriere für Leachables (extrahierbare Stoffe aus dem Material) geschaffen werden kann. Der Applikationseinrichtungs-Ansatz 3 und das Anschlusselement 57 werden von einem Funktionselement in Form einer Überwurfkappe 9 gebildet, die der Fixierung des Verschlussstopfens 23 gegenüber dem Behälter 4, insbesondere am Mundstück 8, dient. Die Überwurfkappe 9 ist als zylindrisches Element mit unterschiedlichen Querschnittsbereichen ausgeführt. Diese ist durch zwei hintereinander angeordnete Teilbereiche charakterisiert, einem ersten Teilbereich 44, welcher den Ansatzbereich für die Applikationseinrichtung bildet und einen zweiten Teilbereich 45, welcher auch als Anschlussbereich bezeichnet wird und die Funktion des Anschlusselementes 57 aufnimmt. Die Überwurfkappe 9 ist im dargestellten Fall als hohlzylindrisches Element unter Ausbildung dieser beiden Teilbereiche ausgeführt. Die Durchgangsöffnung 2 wird vom von der Überwurfkappe 9 umschlossenen Innenraum oder einem Teil dessen gebildet. Der Ansatzbereich für die Applikationseinrichtung ist in Einbaulage betrachtet bezogen auf die Längsachse AL konisch ausgeführt, wobei der Konus sich entlang der Längsachse AL vom Behälter 4 wegerstreckend in Einbaulage ausgeführt ist. Der zweite Teilbereich 45 in Form des Anschlussbereiches ist topfartig ausgeführt, den Behälter 4 im Endbereich 7, insbesondere im Bereich des Mundstückes 8 zu umschließen. Dieser bildet zumindest eine zum Behälter 4 gerichtete Anlagefläche für den Verschlussstopfen 23, die geeignet ist, diesen gegenüber dem Mundstück 8 in Längsrichtung zu fixieren. Die Anlagefläche ist dabei am Innenumfang des Anschlussbereich bildenden Teilbereiches 44 ausgeführt. Vorzugsweise handelt es sich bei den Anlageflächen um zum Behälter 4 in Einbaulage gerichtete Flächen.

Um die Überwurfkappe 9 am Behälter 4 zu fixieren, sind entsprechende Mittel 46 vorgesehen. Diese umfassen beispielhaft an der Überwurfkappe 9 im Anschlussbereich ausgeführte, sich in Richtung des Behälters 4 erstreckende, frei geschnittene Lamellen, die beim Aufsetzen auf das Mundstück 8 aufspreizen und über die Verschluss-Sicherungseinheit 17, insbesondere den Sicherungsring 18 mit diesem verbunden sind. Andere Ausführungen sind denkbar.

Die Überwurfkappe 9 weist im dargestellten Fall einen weiteren koaxial zum Teilbereich 44 angeordneten und um diesen umlaufenden und sich in Richtung der Längsachse AL erstreckenden ringförmigen Bund 47 auf. Dieser ist holzylindrisch ausgeführt. Der Bund 47 erstreckt sich dabei ausgehend vom ersten Teilbereich in Richtung der Längsachse AL unter teilweiser Umschließung unter Einhaltung eines radialen Abstandes zum den Ansatzbereich für die Applikationseinrichtung bildenden Teilbereich 44. Dieser kann hinsichtlich seiner in radialer Richtung gewählten Abmessungen derart ausgeführt sein, geeignet zu sein, mit dem Aufnahmeteil 30 in der Verschluss-Stellung des Verschlusselementes 15 eine Dichtpaarung zu bilden.

Wie bereits ausgeführt, sind vorzugsweise alle Bauteile des Verschlusssystems 1 im Wesentlichen als rotationssymmetrische Bauteile hinsichtlich ihrer Grundkontur ausgeführt und vorzugsweise koaxial zueinander angeordnet.

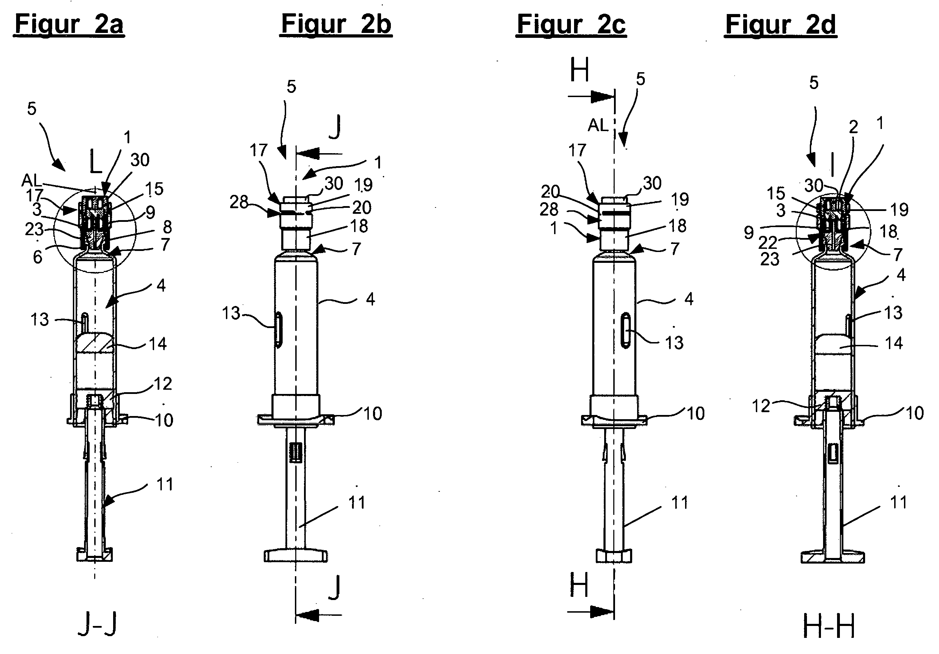

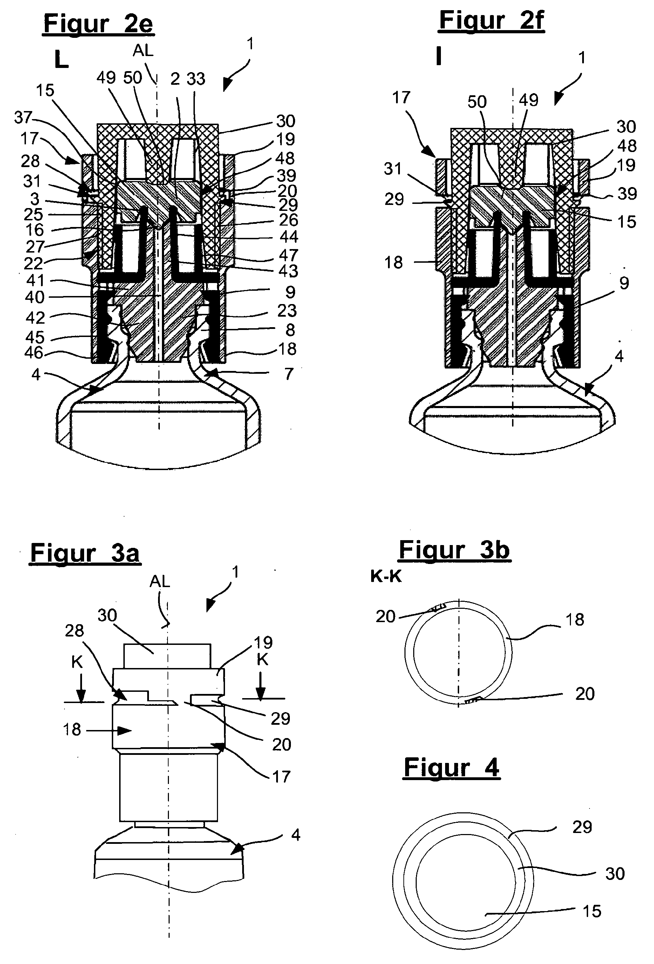

Die Figuren 2a bis 2f zeigen eine weitere vorteilhafte Ausführung eines erfindungsgemäßen Verschlusssystems 1 gemäß einer weiteren Variante einer ersten Ausführung für einen eine Durchgangsöffnung 2 aufweisenden Applikationseinrichtungs-Ansatz 3 in Form eines separaten Funktionselementes zur Kopplung mit einem Behälter 4 am Beispiel einer Spritze 5. Das separate, den Applikationseinrichtungs-Ansatz 3 bildende Funktionselement ist auch hier als Überwurfkappe 9 ausgebildet und dient neben der Ausbildung als Applikationseinrichtungs-Ansatz 3 der Lagefixierung eines die Öffnung eines Behälters 4 verschließenden Verschlussstopfens 23 in Richtung einer Längsachse AL des Verschlusssystems 1, die mit der Längsachse des Behälters 4 zusammenfällt. Zur Charakterisierung der Richtungsangaben wird auf eine theoretische Längsachse AL Bezug genommen, die in Längsrichtung ausgerichtet ist und der Mittenachse der Komponenten in Einbaulage entspricht. In Richtung der Längsachse AL bedeutet dabei in Längsrichtung und in Umfangsrichtung bedeutet um diese. Die Figur 2b verdeutlicht eine Spritze 5 in einer ersten Ansicht, während die Figur 2d eine weitere zweite Ansicht wiedergibt. Die Figur 2a zeigt einen Schnitt J-J gemäß Figur 2b, die Figur 2c einen Schnitt H-H gemäß Figur 2d. Die Figuren 2e und 2f zeigen jeweils das Verschlusssystem 1 mit dem Applikationseinrichtungs-Ansatz 3 im Schnitt gemäß der Details L und I aus den Figuren 2a und 2c.

Der Grundaufbau und die Grundfunktion entsprechen der in den Figuren 1a bis 1f gezeigten Ausführung. Der Unterschied besteht in der Realisierung der Verbindung zwischen Aufnahmeteil 30 und Verschlusselement 15. Diese ist als kraftschlüssige Verbindung 48 in Form einer Pressverbindung ausgeführt. Das Verschlusselement 15 ist derart ausgeführt und dimensioniert, mit dem Innenumfang 33 am Aufnahmeteil 30 eine Presspassung einzugehen. Dazu ist im einfachsten Fall der Außenumfang 37 in Umfangsrichtung mit Übermaß gegenüber dem Innenumfang 33 des Aufnahmeteils 30 ausgebildet. Zur Abstützung der im Funktionszustand verschlossen" des Verschlusssystems 1 am Verschlusselement 15 über die Verbindung durch den Applikationseinrichtungs-Ansatz 3 zum Behälter 4 auftretenden Kräfte ist vorzugsweise das Aufnahmeteil 30 als einseitig geschlossene Kappe ausgeführt, in der das Verschlusselement 15 in Längsrichtung an einer gegen die Bewegungsrichtung beim Lösen ausgerichteten Anschlagfläche 50, die im dargestellten Fall an einem am Innenumfang des geschlossenen Endbereiches des Aufnahmeteils 30 am vom Applikationseinrichtungs-Ansatz 3 weggerichteten Endbereich angeordneten und sich in Längsrichtung in Richtung des Verschlusselementes 15 erstreckenden Vorsprunges 49 zum Anliegen gelangt. Das Aufnahmeteil 30 kann dabei zylindrisch oder wie im dargestellten Fall leicht konisch ausgeführt sein. Der Aufbau der übrigen Komponenten wie Verschlussstopfen 23, Verschluss-Sicherungseinheit 17, insbesondere Sicherungsring 18 und Sicherungsteil 19, Verbindung 22 sowie Überwurfkappe 9, Spritze 5 erfolgt in Analogie zu der in für die Figuren 1a bis 1h beschriebenen Ausführung, weshalb hierzu vollumfänglich auf diese verwiesen wird.

Die Figur 3a zeigt das Verschlusssystem 1 in einer Ansicht gemäß Figur 1c. Erkennbar sind hier die Verbindungsmittel 20, welche zumindest einen, sich in Richtung der Längsachse AL erstreckend verlaufenden und mit seinen voneinander wegweisenden Endbereichen am Sicherungsteil 19 und dem Sicherungsring 18 angelenkten beziehungsweise mit diesen fest verbunden Steg umfassen. Der Steg kann dabei in Längsrichtung zum Sicherungsring 18 oder hier nicht dargestellt zum Sicherungsteil 19 mit sich änderndem Querschnitt ausgeführt sein. Denkbar ist auch eine Querschnittsänderung im Erstreckungsbereich in Längsrichtung in Richtung quer zur Längsachse AL.

Im dargestellten Fall ist ein Verbindungsmittel 20 erkennbar. Denkbar ist beispielsweise auch eine Ausführung mit Anordnung zweier oder mehrerer in Umfangsrichtung um die Längsachse AL beabstandet zueinander angeordneten Verbindungsmitteln 20. Figur 3b verdeutlicht beispielhaft anhand einer Schnittdarstellung K-K gemäß Figur 3a eine Ausführung mit zwei derartigen Verbindungsmitteln 20, die in Umfangsrichtung beabstandet zueinander angeordnet sind und den Sicherungsring 18 und Sicherungsteil 19 in den Bereichen frei von Verbindungsmitteln 20 auf Abstand zueinander halten. Dargestellt ist eine Ansicht in einem Schnitt durch die Verbindungsmittel 20 auf eine am zum Sicherungsteil 19 weisenden Endbereich des Sicherungsringes 18 ausgebildete Fläche. Erkennbar ist, dass die die Verbindungsmittel 20 sich nicht über die gesamte Erstreckung der Anschlussflächen an den die Verschluss-Sicherungseinheit 17 bildenden Teilen - Sicherungsring 18 und Sicherungsteil 19 - erstrecken, sondern bei hier nicht dargestellter analoger Ausführung am Sicherungsteil 19 eine Hinterschneidung für einen am Verschlusselement 15 oder Aufnahmeteil 30 gebildeten Sicherungsvorsprung 29 in Form eines umlaufenden elastischen oder aus Ringsegmenten gebildeten Ringes bildet.

Figur 4 zeigt in einer Ansicht von oben auf ein Aufnahmeteil 30 gemäß Figur 1g die Ausführung mit in Umfangsrichtung um das Aufnahmeteil 30 umlaufend ausgeführten Sicherungsvorsprung 29. Dieser ist derart ausgeführt, in der ersten Funktionsstellung des Verschlusselementes 15 im Funktionszustand "geschlossen" innerhalb der Erstreckung des Verbindungsbereiches 28 in den zwischen Sicherungsteil 19 und Sicherungsring 18, insbesondere den zueinander weisenden Flächen dieser gebildeten Zwischenraum einzugreifen.

In einer hier nicht dargestellten alternativen Ausführung kann der Sicherungsvorsprung 29 auch in Teilbereichen Vorsprünge aufweisen, die direkt hinter den zumindest einen Steg oder Stege umfassenden Verbindungsmitteln 20 zum Liegen kommen und beim Abdrehen des Aufnahmeteils 30 die Verbindungsmittel 20, insbesondere Stege kappen

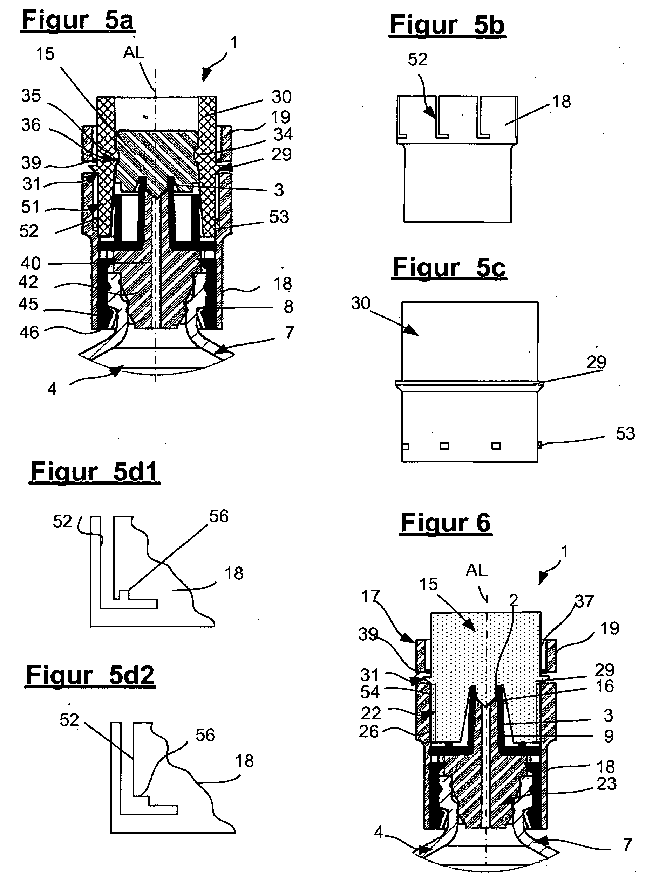

Die Figur 5a zeigt anhand einer Ansicht gemäß Figur 1e beispielhaft eine weitere Ausbildung einer eine Relativbewegung zulassenden Verbindung als formschlüssige Verbindung 51 zwischen Verschlusselement 15 beziehungsweise dem dieses tragenden Aufnahmeteil 30 und einem Anschlussteil, hier ebenfalls in Form des Sicherungsringes 18. Der Grundaufbau - ausgenommen die Verbindung zwischen Anschlussteil in Form des Sicherungsringes 18 und Aufnahmeteil 30 - erfolgt in Analogie zu der in den Figuren 1a bis 1h beschriebenen Ausführung, weshalb diesbezüglich auf diese verwiesen wird.

Die Verbindung 51 wird als formschlüssige Verbindung über am Aufnahmeteil 30 am Außenumfang angeordnete Vorsprünge 53, die in dafür vorgesehene Ausnehmungen 52 am Sicherungsring 18 eingreifen, ausgeführt. Der Formschluss in Längsrichtung betrachtet wird dabei durch die Ausführung der Ausnehmungen 52 mit einem in Längsrichtung erstreckenden ersten randoffenen Teilbereich und einem sich in Umfangsrichtung erstreckenden daran anschließenden weiteren Teilbereich, wobei die Formschlusselemente in Form der Vorsprünge 53 in diese Ausnehmungen 52 eingreifen und bei Montage in diesen in die erste Funktionsstellung des Verschlusselementes geführt werden, gebildet. Die zweite, hier nicht dargestellte Funktionsstellung (Spritzschutz- und/oder Entlüftungsstellung) wird über Drehen des Verschlusselements 15 erreicht, wobei die Vorsprünge 53 entlang der Ausnehmungen 52 in hierfür vorgesehene Rastpositionen 56 in den Ausnehmungen 52 eingerastet werden. Zur Applikation des Produkts werden die Vorsprünge 53 des Verschlusselements aus diesen Rastpositionen 56 bewegt und durch Herausführen der Vorsprünge 53 aus den Ausnehmungen 52 wird das Verschlusselement 15 vollständig vom Applikationseinrichtungs-Ansatz 3 getrennt.

Die Figur 5b zeigt beispielhaft eine mögliche Ausbildung einer durch die Ausnehmungen 52 gebildeten Führung für die Vorsprünge 53 am Aufnahmeteil 30 entsprechend der Ausführung in Figur 5a. Die Ausnehmungen erstrecken sich dabei vom in Einbaulage zum Sicherungsteil 19 weisenden Endbereich des Sicherungsringes 18 in Richtung der Längsachse zur Realisierung eines eine Relativbewegung zwischen Verschlusselement 15 beziehungsweise Aufnahmeteil 30 und Anschlussteil in Form des Sicherungsringes 18 zulassenden Verbindungsbereiches in Längsrichtung des Verschlusssystems 1. Erkennbar ist hier der in Längsrichtung verlaufende erste Teilbereich und der sich daran in Umfangsrichtung erstreckend verlaufende zweite Teilbereich. Der zweite Teilbereich bildet dabei den Verbindungsbereich, welcher einen Formschluss in Längsrichtung ermöglicht. Der erste Teilbereich dient der Relativbewegung.

Ist der Verlauf der Ausnehmung und damit der Verbindungsbereich hier eindeutig in einen in Längs- und Umfangsrichtung wirkenden Teilbereich unterteilt, ist es ebenfalls denkbar, auch den ersten Teilbereich zumindest mit einer Richtungskomponente in Umfangsrichtung verlaufend auszubilden.

In einer vorteilhaften Weiterbildung ist eine Rastposition 56 in der Führung bildenden Ausnehmung 52 vorgesehen. Die Figuren 5d1 und 5d2 verdeutlichen dazu zwei mögliche Ausführungen der Ausnehmung 52 anhand eines Ausschnittes aus dem Verbindungsbereich bildenden Teilbereich des Sicherungsringes 18. Die Rastposition 56 wird in beiden Fällen am in Umfangsrichtung ausgerichteten Teilbereich der Ausnehmung 52 ausgebildet, wobei entsprechend Figur 5d1 die Ausführung beabstandet zum vertikal ausgerichteten Bereich erfolgt, während in Figur 5d2 eine Möglichkeit wiedergegeben ist, bei der die Rastposition 56 in Form einer zum vertikal ausgerichteten Teilbereich der Ausnehmung 52 randoffenen Ausnehmung gebildet wird.

Figur 5c zeigt die Vorsprünge 53 am Aufnahmeteil 30. Diese sind derart angeordnet und ausgeführt, geeignet zu sein, in die Ausnehmungen 52 am Sicherungsteil 18 einzugreifen. Die Anordnung am Außenumfang des Aufnahmeteils 30 erfolgt in Abhängigkeit der Stellung des Aufnahmeteils 30 in Verschluss-Stellung des Verschlusselementes 15 und der Anordnung der zur Erzielung des Formschlusses erforderlichen Ausnehmung 52, insbesondere des in Umfangsrichtung ausgerichteten Teilbereiches dieser.

Die Vorsprünge 53 sind ferner in Einbaulage im Verschlusssystem 1 im zum Applikationseinrichtungs-Ansatz 3 gerichteten Endbereich angeordnet.

Figur 6 verdeutlicht beispielhaft ein erfindungsgemäßes Verschlusssystems 1 gemäß einer zweiten Ausführung mit direkter Verbindung zwischen Verschlusselement 15 und Anschlussteil, insbesondere Sicherungsring 18 in einem Axialschnitt in Analogie zu den Ausführungen gemäß der Figuren 1 und 2. Der Grundaufbau von Verschlusstopfen 23, Überwurfkappe 9 frei von einem Teilbereich 47, Verschluss-Sicherungseinheit 17, Sicherungsring 18, Sicherungsteil 19, Verbindungselement 20 sowie Formschluss 31 entspricht dem in den vorgenannten Figuren beschriebenen, weshalb hier nur darauf verwiesen wird. Die eine Relativbewegung zulassende Verbindung 22 wird hier direkt zwischen Verschlusselement 15 und Anschlussteil in Form des Sicherungsringes 18 gebildet. Die dazu erforderlichen und miteinander in Wirkverbindung bringbaren Verbindungsmittel sind am Außenumfang des Verschlusselementes 15 und Innenumfang des Sicherungsringes 18 angeordnet. Im dargestellten Fall ist diese beispielhaft als Schraubverbindung ausgeführt, welche in der Verschluss-Stellung des Verschlusselementes 15 gegenüber dem Applikationseinrichtungs-Ansatz 3 wiedergegeben ist. Das Verschlusselement 15 weist dazu am Außenumfang ein Außengewinde 54 auf, welches mit einem Innengewinde 26 am Sicherungsring 18 in Eingriff bringbar ist.

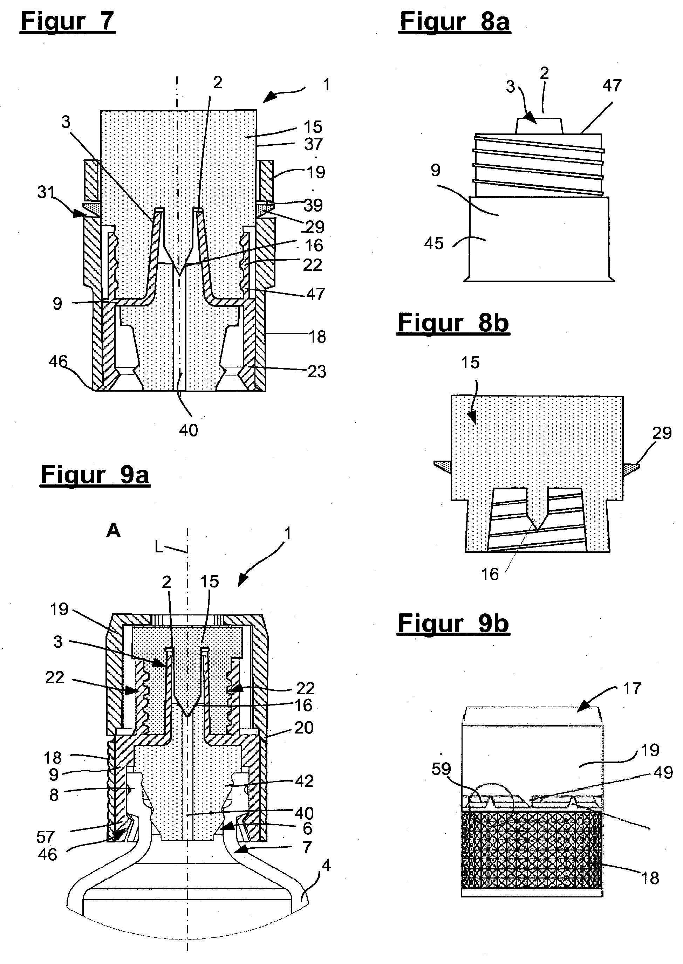

Demgegenüber verdeutlicht Figur 7 ein erfindungsgemäßes Verschlusssystem 1 gemäß einer zweiten Ausführung mit direkter Verbindung zwischen Verschlusselement 15 und Anschlussteil, hier beispielhaft in Form einer den Applikationseinrichtungs-Ansatz 3 bildenden Überwurfkappe 9 in einem Axialschnitt.

Zur Realisierung der Verbindung zwischen Verschlusselement 15 und Applikationseinrichtungs-Ansatz 3, insbesondere Überwurfkappe 9, weist diese einen weiteren, in Analogie zu der in den Figur 1a bis 1h dargestellten Ausführung als ringförmig umlaufender und sich in Längsrichtung erstreckender Bund ausgeführten Teilbereich 47 auf, der derart ausgeführt und angeordnet ist, geeignet zu sein, Verbindungsmittel zur Realisierung einer eine Relativbewegung in Längsrichtung zulassenden Verbindung 22 zwischen Verschlusselement 15 und Überwurfkappe 9 zu tragen, die mit dazu komplementären Verbindungsmitteln am Verschlusselement 15 in Wirkverbindung bringbar sind. Bei der in Figur 7 dargestellten Ausführung wird die eine Relativbewegung ermöglichende Verbindung 22 als kraftschlüssige Verbindung in Form einer Schraubverbindung ausgeführt, wozu das Verschlusselement an einem in Umfangsrichtung verlaufenden Teilbereich seines Außenumfanges ein Außengewinde trägt, welches mit einem an der Überwurfkappe 9, insbesondere dem Teilbereich 47 angeordneten Innengewinde in Eingriff bringbar ist.

Die Figuren 8a und 8b dienen der Verdeutlichung einer zur Ausführung in Figur 7 alternativen Zuordnung von Außen- und Innengewinde zu den Bauteilen Überwurfkappe 9 und Verschlusselement 15. In diesem Fall ist das Verschlusselement 15 mit einem, ein Innengewinde tragenden Teilbereich ausgeführt, während die Überwurfkappe 9, insbesondere im Teilbereich 47 mit einem ein Außengewinde tragenden Bereich ausgeführt ist.

Bei den Ausführungen gemäß der Figuren 6 bis 8 wird der in Richtung der Längsachse AL wirksame, zumindest mittelbar zwischen Verschlusselement 15 und Sicherungsteil 19 gebildeten Formschluss 31 in Analogie zu den Ausführungen der Figuren 1 bis 3 erzeugt, wobei im Unterschied dazu der Sicherungsvorsprung 29 direkt am Verschlusselement 15, insbesondere dem Außenumfang 37 sich in Umfangsrichtung um diesen erstreckend angeordnet ist. Für diesen gelten die gleichen Ausführungen wie bisher für den Sicherungsvorsprung am Aufnahmeteil 30 erläutert.

Die Figur 9a zeigt anhand einer Schnittdarstellung eine Ausführung gemäß Figur 7 mit alternativer Ausführung der Verbindung von Sicherungsring 18 und Sicherungsteil 19. Das Sicherungsteil 19 ist als Sicherungskappe 19 ausgeführt und umschließt dabei zumindest einen Teilbereich des Applikationseinrichtungs-Ansatzes 3 sowie das Verschlusselement 15. Eine vorteilhafte Ausbildung der Verschluss-Sicherungseinheit 17 ist beispielhaft in Figur 9b wiedergegeben. Die Sicherungskappe 19 und der Sicherungsring 18 bilden zwischen sich einen Ringspalt und sind über eine Mehrzahl von Verbindungsstegen 59 und 60 miteinander verbunden. Diese können alternierend oder in einer anderen gewählten Anordnung in Umfangsrichtung zueinander angeordnet sein, wobei die Anzahl der Verbindungsstege 59 und 60 voneinander abweichen kann. Im dargestellten Fall sind die zum Trennen der Verbindung vorgesehenen und eine Sollbruchstelle bildenden Verbindungsstege 59 mit sich in Richtung zum Sicherungsring 18 verjüngendem Querschnitt ausgeführt, während die Druck aufnehmenden Stege mit sich vom Sicherungsring 18 zur Sicherungskappe hin verjüngendem Querschnitt ausgeführt sind. Die Sicherungskappe umschließt dabei das Verschlusselement 15 in Umfangsrichtung über einen wesentlichen Teil von dessen Erstreckung und der Erstreckung der Verbindung 22 in Längsrichtung des Behälters 4 betrachtet.

Andere, hier nicht dargestellte Ausführungen sind denkbar. So können die Verbindungsmittel auch die Sicherungskappe mit dem Sicherungsring 18 verbindende Verbindungsstege umfassen, die durch die Lyophilisatorplatte ausgeübte vertikal auf die Verschluss-Sicherungseinheit 17 wirkende Kraft zum vollständigen Verschließen des Behältnisses 4 ohne Bruch kompensieren. Zwischen jeweils zwei einander benachbarten Verbindungsstegen kann dazu optional jeweils ein Distanzteil zur Druckaufnahme vorgesehen sein, das an der Sicherungskappe fest angeschlossen ist und dessen freies Ende dem Sicherungsring 18 mit geringem Abstand gegenübersteht. Denkbar ist auch die umgekehrte Zuordnung.

Die Verbindungsstege bilden zugleich eine Sollbruchstelle, die es ermöglicht, bei gewünschtem Gebrauch, insbesondere Anschluss der entsprechenden Applikationseinrichtung an den Behälter 4 diese ohne weiteres aufzubrechen.

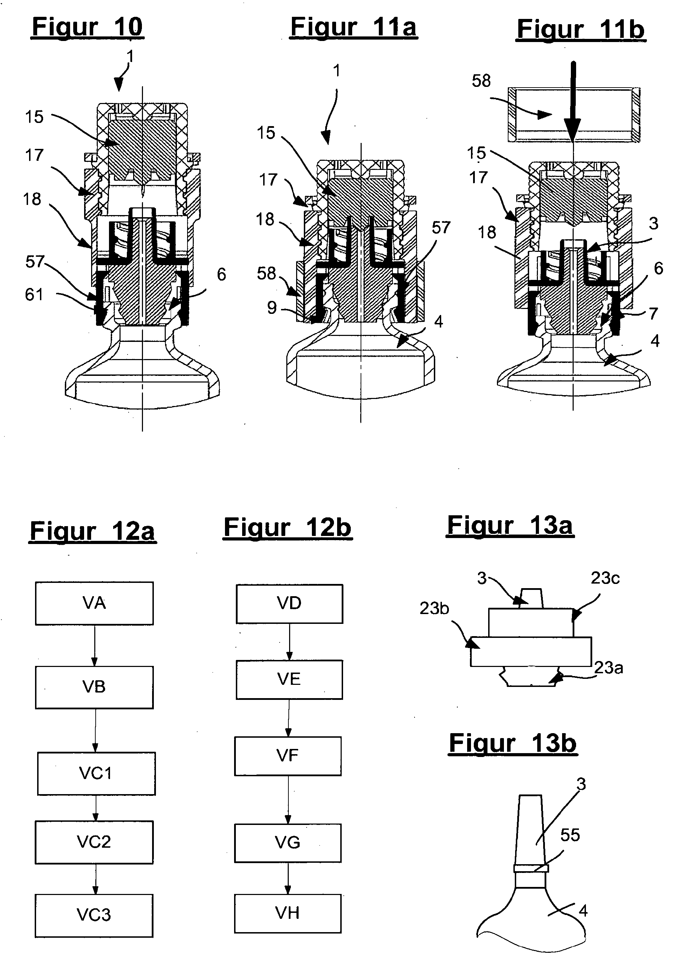

Die Figur 10 zeigt beispielhaft anhand einer Ansicht gemäß Figur 2f die erste Montagestellung des Verschlusssystems 1 gegenüber dem Behälter 4 und die Positionierung der Einzelkomponenten des Verschlusssystems 1 in dieser Montagestellung zueinander, wie diese in besonders vorteilhafter Anwendung in einem Lyophilisator während der Lyophilisation eingenommen werden kann. Das Verschlusssystem 1 wurde vorher zu einer Baueinheit konfektioniert und vorzugsweise bereits sterilisiert.

Die Anordnung des Verschlusssystems 1 erfolgt koaxial zur Längsachse und ist durch eine, eine Verbindung der Öffnung 6 des Behälters 4 zur Umgebung zulassende Positionierung der Einzelkomponenten des Verschlusssystems 1 zueinander charakterisiert. In dieser ersten Montagestellung besteht eine Verbindung zwischen der Öffnung 6 und der Umgebung, d.h. die Öffnung 6 am Behälter 4 ist nicht dichtend verschlossen. Das Verschlusselement 15 ist in dieser Montagestellung frei von einer dichtenden Anordnung des Applikationseinrichtungs-Ansatzes 3. Dies wird durch die Positionierung des Verschlusselementes 15 im vorkonfektionierten Verschlusssystem 1 gegenüber der Verschluss-Sicherungseinheit 17 und die Positionierung der Verschluss-Sicherungseinheit 17 gegenüber dem Anschlusselement 57 realisiert. Die Verschluss-Sicherungseinheit 17 ist in dieser Stellung gegenüber dem Anschlusselement 57 in Längsrichtung betrachtet derart positioniert, dass diese das Anschlusselement 57 nur auf einem Teilbereich seiner Erstreckung in Längsrichtung umschließt, d.h. ist nicht vollständig auf das Anschlusselement 57 aufgeschoben. Die Positionierung des in Einbaulage später behälterseitigen Endbereichs der Verschluss-Sicherungseinheit 17 gegenüber dem Anschlusselement 57 kann durch Positionierhilfen am Innenumfang des Sicherungsringes 18 der Verschluss-Sicherungseinheit 17 und/oder am Außenumfang des Anschlusselementes 57 realisiert werden. Im einfachsen Fall sind diese Positionierhilfen als Vorsprünge ausgeführt, die mit der Gegenfläche am jeweils anderen Element kraftschlüssig oder mit entsprechend komplementären Mitteln formschlüssig zusammenwirken.

Die Positionierung des Anschlusselementes 57 gegenüber dem Behälter 4, insbesondere der Öffnung 6 im Endbereich 7 erfolgt in der ersten Montagestellung in einer ersten Position am Endbereich 7 des Behälters 4. Diese erste Position wird im dargestellten Fall durch das Zusammenwirken der Mittel 46 am Anschlusselement 57 und entsprechend komplementär ausgeführten und zum Zusammenwirken mit diesen geeigneten Vorsprüngen und/oder Ausnehmungen, vorgesehen am Endbereich 7 des Behälters 4 realisiert und ist mit 61 bezeichnet. In dieser Stellung ist die Öffnung 6 dadurch nicht durch den Verschlussstopfen 23 verschlossen. Bei einer Ausführung des Verschlusssystems 1 gemäß Figur 2f und allen Ausführungen, bei welchem das Anschlusselement 57 zur Fixierung des Verschlusssystems 1 am Behälter 4 von einer den Applikationseinrichtungs-Ansatz 3 bildenden Überwurfkappe 9 gebildet wird, ist die Überwurfkappe 9 in der ersten Montagestellung gegenüber der eigentlichen Verschlussstellung relativ zum Behälter 4 in Längsrichtung versetzt angeordnet. Damit ändert sich auch die Lage des an dieser ausgeführten Applikationseinrichtungs-Ansatzes 3 in Längsrichtung gegenüber dem Behälter 4. Durch die Positionierung der Verschluss-Sicherungseinheit 17 relativ zur Überwurfkappe 9 wird ein, eine Verbindung zur Umgebung zulassender Abstand zwischen Öffnung des Applikationseinrichtungs-Ansatzes 3 und dem Verschlusselement 15 realisiert.

Die erste Montagestellung des Verschlusssystems 1 am Behälter 4 ist somit durch eine erste Position, vorzugsweise erste Rastposition des Anschlusselementes 57 am Behälter 4 und einer vordefinierten Lage von Verschluss-Sicherungseinheit 17 und des mit dieser gekoppelten Verschlusselementes 15 in Längsrichtung relativ zum Anschlusselement 57 charakterisiert.

Die Mittel 46, welche am vom Applikationseinrichtungs-Ansatz 3 wegweisenden Endbereich vorgesehen sind, dienen hier im Zusammenwirken mit komplementär ausgeführten Mitteln am Behälter der Lagefixierung in dieser ersten Montagestellung.

Die Verschluss-Sicherungseinheit 17 ist in dieser ersten Montagestellung gegenüber dem Anschlusselement 57, insbesondere der Überwurfkappe 9, in einer von der Verschlussstellung abweichenden Stellung, welche durch eine Lage des Verschlusselementes 15 gegenüber dem Applikationseinrichtungs-Ansatz 3 charakterisiert ist, die frei von einem dichtenden Verschluss ist, angeordnet. Diese Stellung ist durch die gegenüber der Verschlussstellung in Längsrichtung des Verschlusssystems 1 betrachtet versetzte Position der Verschluss-Sicherungseinheit 17 gegenüber dem Anschlusselement 57 charakterisiert.

Die Verschluss-Sicherungseinheit 17, insbesondere der Sicherungsring und damit gekoppelt auch das Verschlusselement 15 sind somit in Längsrichtung des Verschlusssystems 1 gegenüber dem Behälter 4, insbesondere dem Anschlusselement 57 verschoben. Vorzugsweise kann dafür eine Rastposition für die Verschluss-Sicherungseinheit 17 am Anschlusselement 57 vorgesehen werden, welche beispielsweise über Formschlusselemente realisiert werden kann oder sich aufgrund der Auslegung von Anschlusselement 57 und Sicherungsring 18 ergibt. In der ersten Montagestellung können dadurch die bei Lyophilisierung entstehenden Dämpfe abgeleitet werden.

Die Verschluss-Sicherungseinheit 17 wird dann in Richtung Behälter 4 in Längsrichtung verschoben, wobei eine Relativbewegung der Verschluss-Sicherungseinheit 17 gegenüber der Überwurfkappe 9 und damit dem Anschlusselement 57 erfolgt, bei welcher das Verschlusselement 15 den Applikationseinrichtungs-Ansatz 3 abdichtet und gleichzeitig die Überwurfkappe 9 und damit das Anschlusselement 57 in die endgültige, als zweite Montagestellung bezeichnete Verschlussstellung verbracht werden, wie in der Figur 2f dargestellt. Es versteht sich, dass zwischen erster und zweiter Montagestellung auch Zwischenstellungen möglich sein können.

Bei der Ausführung gemäß Figur 2f und Figur 10 erfolgt die Fixierung des Verschlusssystems 1 am Behälter 4 über die Ausgestaltung des Anschlusselementes 57, insbesondere den behälterseitigen Endbereich der Überwurfkappe 9 und die Dimensionierung und Ausgestaltung des zum Behälter 4 weisenden Endbereiches des Sicherungsringes 18. Vorzugsweise wird das Anschlusselement 57 gegenüber dem Behälter 4 durch den endseitigen Bereich des Sicherungsringes 18 verspannt. Um eine genügend große Flächenpressung erzeugen zu können, ist eine bestimmte Erstreckung der miteinander in Wirkverbindung zu bringenden Flächenbereiche von Sicherungsring 18 und Anschlusselement 57 in Längsrichtung des Verschlusssystems 1 vorzusehen.

Die Figuren 11a und 11b zeigen eine besonders vorteilhafte Weiterentwicklung mit einem Spannring 58 zum Verspannen des Sicherungsringes 18 mit dem Anschlusselement 57 beziehungsweise dem Behälter 4, wodurch eine besonders kurze Bauweise für das Verschlusssystem 1 in Längsrichtung und damit auch im Zusammenwirken mit einem Behälter 4 realisiert werden kann.

Figur 11a zeigt die Weiterentwicklung eines Verschlusssystems 1 am Beispiel des in den Figuren 2 beschriebenen Verschlusssystems 1 in einer Ansicht gemäß Figur 2f im Zusammenwirken mit dem Behälter 4 in seiner zweiten Montagestellung, welche durch den dichten Verschluss des Behälters 4 charakterisiert ist. Der Grundaufbau und die Grundfunktion des Verschlusssystems 1 entsprechen den in den Figuren 2 dargelegten Ausführungen, ausgenommen der Aufbringung der Fixierkraft für das Verschlusssystem 1 am Behälter 4. Bezüglich der grundlegenden Ausführung und Funktionsweise wird daher auf die Ausführung zu diesen Figuren verwiesen. Für gleiche Elemente sind gleiche Bezugszeichen verwendet. Ferner kann die Ausgestaltung des Verschlusssystems 1 - ausgenommen des diese Ausführung charakterisierenden Unterscheidungsmerkmals der Verspannung durch einen separaten Spannring 58 - auch, wie hier nicht dargestellt, analog zu den anderen beschriebenen Ausführungen erfolgen.

Im dargestellten Fall wird die erforderliche Kraft zur Fixierung des Anschlusselementes 57 gegenüber dem Behälter 4 durch einen Spannring 58 aufgebracht. Dieser umgreift in der Verschlussstellung den Sicherungsring 18 in Umfangsrichtung vollständig. Denkbar wäre auch der Einsatz eines, den Sicherungsring 18 in Umfangsrichtung nur teilweise, umschließenden Klemmringes. Da die Verspannung nicht mehr allein über die Dimensionierung des Sicherungsringes 18 erzeugt werden muss, kann dieser in seiner Erstreckung in Längsrichtung verkürzt ausgeführt werden. Dies gilt damit auch für das Anschlusselement 57 und bei Ausbildung dessen an der Überwurfkappe 9 auch für diese.

Die Figur 11b zeigt in Analogie zur Figur 10 das Verschlusssystem in der ersten Montagestellung mit separatem Spannring, dessen Montagerichtung mit Pfeil verdeutlicht ist. Der Spannring 58 wird jedoch auf das Verschlusssystem 1 erst nachdem dieses bereits in der zweiten Montagestellung gegenüber dem Behälter ist, aufgeschoben. Dies kann noch in einem Lyophilisator oder aber auch erst nach Herausnahme erfolgen.

Zeigen die Figuren 1 bis 8 Ausführungen mit einem Applikationseinrichtungs-Ansatz 3, welcher von einer Überwurfkappe 9 gebildet wird, verdeutlicht Figur 13a beispielhaft eine alternative Ausführung des Applikationseinrichtungs-Ansatzes 3 vom Verschlussstopfen 23. Für diese Ausführung ist der Verschlussstopfen 23 derart ausgeführt und angeordnet, geeignet zu sein, neben der Ausbildung einer am Außenumfang in Umfangsrichtung verlaufenden Dichtfläche auch die form- und/oder kraftschlüssige Verbindung mit dem Mundstück 8 zu realisieren. Dies wird über entsprechende Formgebung realisiert, indem beispielsweise der Verschlussstopfen 23 einen Teilbereich 23a aufweist, der geeignet ist, zumindest über einen Teilbereich dichtend am Innenumfang des Mundstückes 8 anzuliegen und ferner einen weiteren Teilbereich 23b, der senkrecht zur Längsachse AL das Mundstück 8 umgreifend ausgeführt ist. Der Verbindungsbereich wird von einem ringförmig den Anschlussbereich beabstandet umschließenden weiteren Teilbereich 23c gebildet. Die Mittel zur Realisierung der Verbindung 22 mit dem Verschlusselement 15 können dabei in Abhängigkeit der Ausbildung der dazu komplementären Mittel am Verschlusselement am Innen- oder Außenumfang des Teilbereiches 23c angeordnet sein. Neben dieser Ausführung besteht eine weitere Möglichkeit in der Ausführung eines Gewindes am Behälter 4. In diesem Bereich braucht der Verschlussstopfen 23 nur auf- bzw. eingeschraubt werden.

Figur 13b zeigt eine Ausführung mit Ausbildung des Applikationseinrichtungs-Ansatzes 3 durch Ausformung am Behälter 4. Zur Fixierung des Verschlusssystems 1 ist ein Haltering 55 auf diesen aufgeschoben und form- und/oder kraftschlüssig an diesem befestigt.

Figur 12a verdeutlicht in schematisiert vereinfachter Darstellung beispielhaft den Ablauf eines Verfahrens zur Herstellung einer Spritze 5. In einem ersten Verfahrensschritt VA wird das Verschlusssystem 1 vormontiert. Im Verfahrensschritt VB wird das gesamte Verschlusssystem 1 sterilisiert und um Verfahrensschritt VC mit einem Behälter 4 in entsprechender Ausgestaltung mit einem Kolben 11 unter Ausbildung der Spritze 5 komplettiert. Dabei wird vorzugsweise in VC1 das Verschlusssystem derart gegenüber dem Behälter positioniert, dass die Überwurfkappe 9 das Mundstück 8 umgreift und der Verschlussstopfen in der Öffnung 6 angeordnet ist. Die Verschluss-Sicherungseinheit 17 befindet sich in der ersten Montagestellung. In dieser ist das Verschlusselement 15 aus der dichtenden Position gegenüber dem Applikationseinrichtungs-Ansatz 3 entfernt. In dieser Stellung erfolgt in VC2 die Lyophilisation, während in VC3 die Verschluss-Sicherungseinheit 17 gegenüber dem Anschlusselement 57 in die zweite Montagestellung durch Vertikalbewegung verbracht wird und in dieser das Anschlusselement 57 gegenüber dem Behälter 4 und damit den Applikationseinrichtungs-Ansatz 3 fixiert und das Verschlusselement 15 in eine, den Applikationseinrichtungs-Ansatz 3 abdichtende Stellung verbringt.

Figur 12b gibt in schematisiert vereinfachter Darstellung beispielhaft den Ablauf eines Verfahrens beim Öffnen des Verschlusssystems 1 wieder. Im Verfahrensschritt VD liegt das Verschlusssystem 1 im Funktionszustand "verschlossen" vor. Der Applikationseinrichtungs-Ansatz 3 ist durch das Verschlusselement 15 druck-, medien- und mikrobiell dicht verschlossen. Im Verfahrensschritt VE erfolgt die Trennung der Verbindung zwischen Sicherungsteil 19 und Sicherungsring 18 unter Auflösung des in Längsrichtung wirkenden Formschlusses 31 zwischen Verschlusselement 15 beziehungsweise des diesen aufnehmenden Aufnahmeteils 30 und dem Sicherungsteil 19. Die Trennung kann durch Auslösen der Relativbewegung zwischen Verschlusselement 15 beziehungsweise Aufnahmeteil 30 und Anschlussteil ausgelöst werden. In VF wird das Verschlusselement 15 in die zweite Funktionsstellung, welche einen Spritzschutz und eine Entlüftung gewährleistet, verbracht. Dazu wird das Verschlusselement 15 beziehungsweise das Aufnahmeteil 30 entsprechend der erforderlichen Bewegungsrichtung innerhalb de Verbindung bewegt und in diese Funktionsstellung verbracht. Nach erfolgtem Entlüften kann das Verschlusselement in VG vollständig vom Applikationseinrichtungs-Ansatz 3 wegbewegt werden und in VH eine Applikationseinrichtung an den Applikationseinrichtungs-Ansatz 3 angeschlossen werden.

## Patentansprüche

1. Verschlusssystem (1) für einen sich entlang einer theoretischen Längsachse (AL) erstreckenden, eine Durchgangsöffnung (2) aufweisenden und zum Anschluss einer Applikationseinrichtung ausgebildeten Applikationseinrichtungs-Ansatz (3) an einem Behälter (4) für pharmazeutische Präparate,
umfassend
- ein Verschlusselement (15) mit zumindest einem, eine Dichtfläche (16) aufweisenden Bereich zur Abdichtung der Durchgangsöffnung (2) des Applikationseinrichtungs-Ansatzes (3),
- ein hülsenartiges Anschlusselement (57) zur kraft- und/oder formschlüssigen Verbindung mit dem Behälter im applikationseinrichtungsseitigen Endbereich;
- eine koaxial zum Anschlusselement (57) angeordnete und dieses in Umfangsrichtung umschließende Verschluss-Sicherungseinheit (17) mit einem Sicherungsring (18) und einem mit dem Sicherungsring (18) über Verbindungsmittel (20) abtrennbar unter irreversibler Zerstörung dieser verbundenes Sicherungsteil (19), wobei die Verschluss-Sicherungseinheit (17) ausgelegt ist, zumindest den Endbereich des Applikationseinrichtungs-Ansatzes (3) und das Verschlusselement (15) in Umfangsrichtung um die Längsachse (AL) über einen Teilbereich der Erstreckung in Längsrichtung zu umschließen und durch Relativbewegung in Längsrichtung gegenüber dem Anschlusselement (57) von einer ersten in eine zweite Montagestellung das hülsenartige Anschlusselement (57) am Behälter (4) zu fixieren;
**dadurch gekennzeichnet,**
**dass** das Verschlusselement (15) wenigstens mittelbar mit einem vom Applikationseinrichtungs-Ansatz (3) oder einem diesen tragenden Funktionselement oder dem Sicherungsring (18) gebildeten Anschlussteil lösbar kraft- und/oder formschlüssig verbunden ist, wobei das Verschlusselement (15) in einer als Verschluss-Stellung definierten ersten Funktionsstellung die Durchgangsöffnung (2) am Applikationseinrichtungs-Ansatz (3) druckdicht, mediendicht und mikrobiell dicht verschließt und die form- und/oder kraftschlüssige Verbindung (22, 51) derart angeordnet und ausgeführt ist, geeignet zu sein, nach Abtrennung des Sicherungsteils (19) vom Sicherungsring (18) eine Relativbewegung zwischen den an der Verbindung (22, 51) beteiligten Bauteilen, insbesondere Verschlusselement (15) und Anschlussteil mit einer Richtungskomponente in Richtung der Längsachse (AL) aus der ersten Funktionsstellung (Verschluss-Stellung) in zumindest eine weitere Funktionsstellung (Spritzschutz- und/oder Entlüftungsstellung) unter zumindest teilweiser Aufrechterhaltung dieser Verbindung (22, 51) zu ermöglichen, wobei in der zumindest einen weiteren Funktionsstellung die druck- , medien- und mikrobiell dichte Abdichtung der Durchgangsöffnung (2) am Applikationseinrichtungs-Ansatz (3) durch das Verschlusselement (15) unter Bildung eines zwischen Verschlusselement (15), Applikationseinrichtungs-Ansatz (3) und/oder Anschlussteil gebildeten Zwischenraumes (32) aufgehoben ist.

2. Verschlusssystem (1) nach Anspruch 1,
**dadurch gekennzeichnet,**
**dass** Entlüftungsmittel (21) vorgesehen sind, die wenigstens teilweise in der wenigstens mittelbaren Verbindung (22, 51) zwischen Verschlusselement (15) und Anschlussteil integriert sind und derart angeordnet und ausgeführt sind, geeignet zu sein, zumindest in der zweiten Funktionsstellung des Verschlusselementes (15) die Durchgangsöffnung (2) am Applikationseinrichtungs-Ansatz (3) mit der Umgebung zu verbinden.

3. Verschlusssystem (1) nach Anspruch 1 oder 2,
**dadurch gekennzeichnet,**
**dass** die zumindest mittelbare Verbindung (22, 51) zwischen Verschlusselement (15) und Anschlussteil indirekt von einer Verbindung zwischen einem, das Verschlusselement (15) zumindest in Umfangsrichtung um die Längsachse (AL) betrachtet umschließenden und mit diesem verbundenen Aufnahmeteil (30) und dem Anschlussteil gebildet wird, und die Verbindung zwischen Verschlusselement (15) und Aufnahmeteil (30) als eine der nachfolgend genannten Verbindungen oder eine Kombination aus diesen ausgeführt ist:
- kraftschlüssige Verbindung
- formschlüssige Verbindung
- stoffschlüssige Verbindung
und
ein in Richtung der Längsachse (AL) wirksamer, zumindest mittelbar zwischen Verschlusselement (15) und Sicherungsteil (19) gebildeter Formschluss (31) zwischen Aufnahmeteil (30) und Sicherungsteil (19) gebildet ist,
oder
**dass** die zumindest mittelbare Verbindung (22, 51) zwischen Verschlusselement (15) und Anschlussteil von einer direkten Verbindung von Verschlusselement (15) und Anschlussteil gebildet wird und ein in Richtung der Längsachse (AL) wirksamer, zumindest mittelbar zwischen Verschlusselement (25) und Sicherungsteil (19) gebildeter Formschluss (31) zwischen Verschlusselement (15) und Sicherungsteil (19) gebildet ist.

4. Verschlusssystem (1) nach einem der vorhergehenden Ansprüche,
**dadurch gekennzeichnet,**
**dass** die zumindest mittelbare, eine Relativbewegung zwischen dem Verschlusselement (15) und dem Anschlusselement mit einer Richtungskomponente in Richtung der Längsachse (AL) zulassende Verbindung (22, 51) zumindest über einen Teilbereich mit einem in Richtung der Längsachse (AL) der Bewegungsrichtung zum Lösen des Verschlusselementes (15) entgegengerichtet wirkenden Form- und/oder Kraftschlussverbindung ausgeführt ist.

5. Verschlusssystem (1) nach einem der vorhergehenden Ansprüche,
**dadurch gekennzeichnet,**
**dass** die zumindest mittelbare, eine Relativbewegung zwischen dem Verschlusselement (15) und dem Anschlusselement mit einer Richtungskomponente in Richtung der Längsachse (AL) zulassende Verbindung (22, 51) von einer Schraubverbindung gebildet wird, umfassend einen ein Innengewinde tragenden Teilbereich an einem der miteinander zu verbindenden Bauteile - Verschlusselement (15) beziehungsweise Aufnahmeteil (30) oder Anschlussteil - und einen ein Außengewinde tragenden Teilbereich am jeweils anderen der miteinander zu verbindenden Bauteile - Anschlussteil oder Verschlusselement (15) beziehungsweise Aufnahmeteil (30).

6. Verschlusssystem (1) nach Anspruch 5,
**dadurch gekennzeichnet,**
**dass** wenigstens ein, sich mit zumindest einer Hauptrichtungskomponente parallel zur Längsachse (AL) erstreckender Entlüftungskanal vorgesehen ist, der in das Gewinde eingearbeitet ist oder durch Unterbrechung der Gewindegänge unter Ausbildung von einem Gewinde freier Bereiche an zumindest einem der miteinander zu verbindenden Bauteile - Verschlusselement (15) beziehungsweise Aufnahmeteil (30) oder Anschlussteil -, ausgebildet ist, wobei die Anordnung und Dimensionierung der von einem Gewinde freien Bereiche derart erfolgt, dass die Summe der von einem Gewinde freien Winkelbereiche im Bereich von 5° bis 90°, vorzugsweise 8° bis 60°, besonders bevorzugt 10° und 45° bezogen auf den vollen Umfang 360 beträgt,
und/oder
die weitere Funktionsstellung (Entlüftungsstellung) zwischen Verschlusselement (15) und Anschlusselement als Funktion der Geometrie und/oder Dimensionierung des Gewindes, insbesondere der Gewindesteigung, definiert ist.

7. Verschlusssystem (1) nach einem der Ansprüche 1 bis 4,
**dadurch gekennzeichnet,**
**dass** die zumindest mittelbare, eine Relativbewegung zwischen dem Verschlusselement (15) und dem Anschlusselement mit einer Richtungskomponente in Richtung der Längsachse (AL) zulassende Verbindung (22, 51) als formschlüssige Verbindung ausgeführt ist, umfassend an einem der zu verbindenden Bauteile- Verschlusselement (15) beziehungsweise Aufnahmeteil (30) oder Anschlussteil- angeordnete, in Richtung zum anderen Bauteil gerichtete Vorsprünge, die in am jeweils anderen der miteinander zu verbindenden Bauteile -Anschlussteil oder Verschlusselement (15) beziehungsweise Aufnahmeteil (30 - in randoffene, in Richtung der Längsachse verlaufende und jeweils in einen in Umfangsrichtung ausgerichteten Teilbereich mündende Aussparungen (52) einführbar sind.

8. Verschlusssystem (1) nach einem der vorhergehenden Ansprüche,
**dadurch gekennzeichnet,**
**dass** in der ersten Funktionsstellung zwischen Verschlusselement (15) und Sicherungsteil (19) ein in Richtung der Längsachse (AL) zumindest in einer Richtung wirksamer Formschluss (31) vorgesehen ist und der in Richtung der Längsachse (AL) wirksame, zumindest mittelbar zwischen Verschlusselement (25) und Sicherungsteil (19) gebildete Formschluss (31) im Verbindungsbereich (28) zwischen Sicherungsteil (19) und Sicherungsring (18) derart angeordnet und ausgeführt ist, dass bei Abtrennung des Sicherungsteils (19) der Formschluss aufgehoben ist.

9. Verschlusssystem (1) nach Anspruch 8,
**dadurch gekennzeichnet,**
**dass** das Sicherungsteil (19) und der Sicherungsring (18) über wenigstens ein in Längsrichtung ausgebildetes Verbindungsmittel (20) miteinander verbunden sind und das Sicherungsteil (19) eine zum Sicherungsring (18) weisende Fläche (39) aufweist, die als Anschlagfläche in Längsrichtung für zumindest einen am Verschlusselement (15) oder dem Aufnahmeteil (30) in Umfangsrichtung um diesen ausgebildeten zumindest ringsegmentförmigen elastischen Sicherungsvorsprung (29) in der ersten Funktionsstellung des Verschlusselementes (15) fungiert.

10. Verschlusssystem (1) nach einem der Ansprüche 1 bis 9,
**dadurch gekennzeichnet,**
**dass** das Anschlusselement (57) Mittel (46) zur form-und/oder kraftschlüssigen Kopplung mit dem Behälter (4) aufweist.

11. Verschlusssystem (1) nach einem der Ansprüche 1 bis 10,
**dadurch gekennzeichnet,**
**dass** der der Applikationseinrichtungs-Ansatz (3) hohlzylindrisch ausgeführt ist und zumindest einen konisch ausgeführten Teilbereich aufweist, wobei der Applikationseinrichtungs-Ansatz (1) von einem am Behälter (4) angeformten Bereich gebildet wird und der Sicherungsring (18) direkt oder über einen, den Applikationseinrichtungs-Ansatz (3) in Umfangsrichtung umschließenden Haltering in Umfangsrichtung und in Richtung der Längsachse (AL) am Behälter (4) befestigt ist, oder dass der Applikationseinrichtungs-Ansatz (3) hohlzylindrisch ausgeführt ist und zumindest einen konisch ausgeführten Teilbereich aufweist und
der Applikationseinrichtungs-Ansatz (3) von einem separaten Element gebildet wird oder an einem Bauteil des Verschlusssystems (1) mit ausgebildet ist, welches wenigstens mittelbar form- und/oder kraftschlüssig mit dem Behälter (4) verbunden ist.

12. Verschlusssystem (1) nach Anspruch 11,
**dadurch gekennzeichnet,**
**dass** dieses einen in eine endseitige Öffnung (6) am Behälter (4) einbringbaren, eine Durchgangsöffnung (40) zur Verbindung zwischen Behälterinnenraum und Umgebung aufweisenden Verschlussstopfen (23) mit einer bezogen auf die Längsachse (AL) verlaufenden äußeren Umfangsfläche, welche über einen Teilbereich eine Dichtfläche zur Anlage an einem Teilbereich des Innenumfanges des Behälters (4) im Bereich der endseitigen Öffnung (6) bildet, umfasst,
der Verschlussstopfen über eine diesen umschließende Überwurfkappe (9) am Behälter (4) kraft- und/oder formschlüssig fixiert ist und
der Applikationseinrichtungs-Ansatz (3) von zumindest einem Teilbereich der Überwurfkappe (9) oder einen sich durch die Überwurfkappe (9) erstreckenden Teilbereich am Verschlussstopfen (23) gebildet ist.

13. Verschlusssystem (1) nach Anspruch 12,
**dadurch gekennzeichnet,**
**dass** die Überwurfkappe (9) als hohlzylindrisches Element ausgeführt ist, umfassend zumindest zwei koaxial zueinander angeordnete Teilbereiche, einen ersten Teilbereich (44) zur Ausbildung des Anschlussbereiches des Applikationseinrichtungs-Ansatzes (3), einen zweiten Teilbereich (45) zur Befestigung am Behälter (4), und optional einen dritten Teilbereich (47) zur Anordnung von Verbindungsmitteln zur mittelbaren kraft- und/oder formschlüssigen Verbindung (22, 51) des Verschlusselementes (15) mit einem Anschlussteil bei Ausbildung der Überwurfkappe (9) als Anschlussteil, wobei der optional vorgesehene dritte Teilbereich den ersten Teilbereich wenigstens teilweise über dessen Erstreckung in Richtung der Längsachse (AL) in Umfangsrichtung unter Ausbildung eines Zwischenraumes umschließt und/oder
**dass** das Anschlusselement (57) von der Überwurfkappe (9) gebildet wird, die im Anschlussbereich an den Behälter (4) gegenüber diesem wirksame Rastmittel aufweist.

14. Verschlusssystem (1) nach einem der vorhergehenden Ansprüche,
**dadurch gekennzeichnet,**
**dass** die Verschluss-Sicherungseinheit (17) ausgelegt ist, bei Relativbewegung in Längsrichtung gegenüber dem Anschlusselement (57) von einer ersten in eine zweite Montagestellung das hülsenartige Anschlusselement (57) am Behälter (4) zumindest teilweise zu fixieren.

15. Verschlusssystem (1) nach Anspruch 14,
**dadurch gekennzeichnet,**
**dass** ein den Sicherungsring (18) der Verschluss-Sicherungseinrichtung (17) in der zweiten Montagestellung in Umfangsrichtung im Erstreckungsbereich des Anschlusselementes (57) umschließender Spannring (58) vorgesehen ist, welcher den Sicherungsring und das Anschlusselement (57) gegenüber dem Behälter (4) verspannt.

16. Verschlusssystem (1) nach einem der vorhergehenden Ansprüche,
**dadurch gekennzeichnet,**
**dass** dieses mit dem Applikationseinrichtungs-Ansatz (3) als die Verschluss-Stellung zwischen Verschlusselement (15) und Applikationseinrichtungs-Ansatz (3) einhaltende vormontierte Baueinheit ausgeführt ist.

17. Verwendung eines Verschlusssystems (1) gemäß einem der Ansprüche 1 bis 16 zum Verschließen eines zumindest ein pharmazeutisches Präparat aufnehmenden Behälters (4), insbesondere eines Doppelkammer-Behälters.

18. Behälter (4) zur Aufnahme pharmazeutischer Präparate mit einem an diesem ausgebildeten oder befestigbaren Applikationseinrichtungs-Ansatz (3) mit einem Verschlusssystem (1) gemäß einem der Ansprüche 1 bis 16.

19. Behälter (4) nach Anspruch 18,
**dadurch gekennzeichnet,**
**dass** dieser als Doppelkammer-Behälter mit einem Mundstück (8) zur Ausbildung der Öffnung (6) und zum Anschluss des Applikationseinrichtungs-Ansatzes (3) ausgebildet ist, wobei das Mundstück (8) zumindest zwei komplementär zu den Mitteln (46) am Anschlusselement (57) ausgebildete Mittel zur Realisierung eines Kraft- und/oder Formschlusses umfasst, die in Längsrichtung zueinander versetzt angeordnet sind, um in der ersten Montagestellung des Verschlusssystems (1) das Anschlusselement (57) in einer ersten Lage gegenüber dem Behälter (4) zu halten und in der zweiten Montagestellung des Verschlusssystems (1) das Anschlusselement (57) in einer zweiten Lage gegenüber dem Behälter (4) zu fixieren.

20. Spritze (5) für medizinische Zwecke, umfassend einen Behälter (4) gemäß einem der Ansprüche 18 oder 19.

21. Verfahren zur Montage eines Behälters (4) mit einem Verschlusssystem (1) nach einem der Ansprüche 18 oder 19,
**gekennzeichnet durch folgende Merkmale,**
- Vormontage des Verschlusssystems (1) aus zumindest einem Verschlusselement (15) und Verschluss-Sicherheitseinheit (17) zu einer Baueinheit
- Reinigung und/oder Sterilisation des Verschlusssystems (1)
- Zuordnung zu einem Behälter (4) zur Aufnahme pharmazeutischer Produkte und Verschluss eines endseitigen Bereiches (7) eines pharmazeutische Produkte aufnehmenden Behälters (4) ;
- Verschluss des Behälters (4) **durch** Aufschieben des Verschlusssystems (1).

22. Verfahren nach Anspruch 21,
**dadurch gekennzeichnet,**
**dass**
- das Verschlusssystem (1) zum Behälter vor der Lyophilisierung in eine erste Montagestellung verbracht wird, in welcher das Anschlusselement (57) am die Öffnung (6) aufweisenden Endbereich (7) des Behälters (4) diesen in Umfangsrichtung umschließend in einer ersten Stellung angeordnet wird und die Verschluss-Sicherungseinheit (17) und das mit dieser gekoppelte Verschlusselement (15) in Längsrichtung betrachtet gegenüber dem Anschlusselement (57) in einer Lage frei von einer Abdichtung des Applikationseinrichtungs-Ansatzes (3) positioniert wird und
- das Verschließen des Behälters (4) während oder nach einer Lyophilisation durch Verbringen des Verschlusssystems (1) in eine zweite Montagestellung unter Relativbewegung zumindest einzelner Komponenten des Verschlusssystems (1) zueinander und/oder gegenüber dem die Öffnung (6) beinhaltenden Endbereich (7), insbesondere Verschiebung der Verschluss-Sicherungseinheit (17) und des Verschlusselementes (15) relativ zum Anschlusselement (57) in Längsrichtung unter dichtendem Verschließen des Applikationseinrichtungs-Ansatzes (3) und Verschiebung des Anschlusselementes (57) am Endbereich (7) des Behälters unter Fixierung des Verschlusssystems (1) am Behälter (4) nach der Lyophilisierung in eine zweite Stellung erfolgt.

## Claims

1. A closure system (1) for an applicator projection (3) on a container (4) for pharmaceutical preparations, said applicator projection extending along a theoretical longitudinal axis (AL), having a through-opening (2) and being formed for the connection of an applicator, comprising
- a closure element (15) with at least one region having a sealing surface (16) for sealing the through-opening (2) of the applicator projection (3),
- sleeve-shaped connecting element (57) for a friction-locked and/or interlocking connection with the container in the end region on the applicator side;
- a closure securing unit (17) which is arranged coaxially to the connecting element (57), surrounds said element in the circumferential direction and comprises a securing ring (18) and a securing part (19) which is severably connected to the securing ring (18) via connecting means (20) under irreversible destruction thereof, wherein the closure securing unit (17) is formed to surround at least the end region of the applicator projection (3) and the closure element (15) in the circumferential direction around the longitudinal axis (AL) over a section of the extension in the longitudinal direction, and to fix the sleeve-shaped connecting element (57) to the container (4) by relative movement in the longitudinal direction in relation to the connecting element (57) from a first to a second mounting position;
**characterized in that**
the closure element (15) is at least indirectly detachably connected in a friction-locked and/or interlocking manner to a connecting part formed by the applicator projection (3) or a functional element that carries said applicator projection or the securing ring (18), wherein the closure element (15), in a first functional position defined as the closure position, tightly seals the through-opening (2) on the applicator projection (3) in a pressure-tight, media-tight and microbially-tight manner, and the interlocking and/or friction-locked connection (22, 51) is arranged and formed in such a way that it is capable, after the severing of the securing part (19) from the securing ring (18), of enabling a relative movement between the components involved in the connection (22, 51), especially the closure element (15) and the connecting part, with a directional component in the direction of the longitudinal axis (AL) from the first functional position (closure position) to at least one further functional position (splash-protection and/or ventilation position) under at least partial maintenance of said connection (22, 51), wherein in the at least one further functional position the pressure-tight, media-tight and microbially-tight sealing of the through-opening (2) on the applicator projection (3) is eliminated by the closure element (15) by forming an intermediate space (32) formed between the closure element (15), the applicator projection (3) and/or the connecting part.

2. A closure system (1) according to claim 1,
**characterized in that**
venting means (21) are provided, which are integrated at least partly in the at least indirect connection (22, 51) between the closure element (15) and the connecting part, and are arranged and formed in such a way that they are capable, at least in the second functional position of the closure element (15), to connect the through-opening (2) on the applicator projection (3) to the ambient environment.

3. A closure system (1) according to claim 1 or 2,
**characterized in that**
the at least indirect connection (22, 51) between the closure element (15) and the connecting part is formed indirectly by a connection between a receiving part (30) and the connecting part, said receiving part surrounding the closure element (15) at least in the circumferential direction when seen around the longitudinal axis (AL) and being connected thereto, and the connection between the closure element (15) and the receiving part (30) is formed as one of the following mentioned connections or a combination thereof:
- friction-locked connection;
- interlocking connection;
- materially bonded connection;
and
an interlocking connection (31) is formed between the receiving part (30) and the securing part (19), said interlocking connection being effective in the direction of the longitudinal axis (AL) and being formed at least indirectly between the closure element (15) and the securing part (19),
or
the at least indirect connection (22, 51) between the closure element (15) and the connecting part is formed by a direct connection of the closure element (15) and connecting part, and an interlocking connection (31) is formed between the closure element (15) and the securing part (19), said interlocking connection being effective in the direction of the longitudinal axis (AL) and being formed at least indirectly between the closure element (25) and the securing part (19).

4. A closure system (1) according to one of the preceding claims,
**characterized in that**
the at least indirect connection (22, 51), which permits a relative movement between the closure element (15) and the connecting element with a directional component in the direction of the longitudinal axis (AL), is formed at least over a section with an interlocking and/or friction-locked connection which in the direction of the longitudinal axis (AL) acts in the direction opposite of the direction of movement for releasing the closure element (15).

5. A closure system (1) according to one of the preceding claims,
**characterized in that**
the at least indirect connection (22, 51), which permits a relative movement between the closure element (15) and the connecting element with a directional component in the direction of the longitudinal axis (AL), is formed by a threaded connection, comprising a section carrying an internal thread on one of the components to be connected, i.e. the closure element (15) or the receiving part (32) or connecting part, and a section carrying an external thread on the respective other components to be connected to each other, i.e. the connecting part or closure element (15) or receiving part (30).

6. A closure system (1) according to claim 5,
**characterized in that**
at least one venting channel is provided, which extends with at least one main directional component parallel to the longitudinal axis (AL) and which is incorporated in the thread or is formed by interruption of the thread grooves by forming a thread-free region on at least one of the components to be connected, i.e. the closure element (15) or the receiving part (30) or the connecting part, wherein the arrangement and dimensioning of the thread-free regions occur in such a way that the sum total of the angular ranges that are free from a thread is in the region 5° to 90°, preferably 8° to 60°, more preferably 10° and 45° relating to the full circumference 360,
and/or
the further functional position (venting position) between the closure element (15) and connecting element is defined as a function of geometry and/or dimensioning of the thread, especially the thread pitch.

7. A closure system (1) according to one of the claims 1 to 4,
**characterized in that**
the at least indirect connection (22, 51), which permits a relative movement between the closure element (15) and the connecting element with a directional component in the direction of the longitudinal axis (AL), is formed as an interlocking connection, comprising projections which are arranged on one of the components to be connected, i.e. closure element (15) or receiving part (30) or connecting part, are oriented in the direction of the other component and can be inserted into open-ended recesses (52) in components to be connected to the respective other of the components to be connected, i.e. connecting part or closure element (15) or receiving part (30), said recesses extending in the direction of the longitudinal axis and respectively opening into a section oriented in the circumferential direction.

8. A closure system (1) according to one of the preceding claims,
**characterized in that**
an interlocking connection (31), which is effective in the direction of the longitudinal axis (AL) at least in one direction, is provided in the first functional position between the closure element (15) and the securing part (19), and said interlocking connection (31), which is effective in the direction of the longitudinal axis (AL) and is formed at least indirectly between the closure element (25) and the securing part (19), is arranged and formed in the connecting region (28) between the securing part (19) and the securing ring (18) in such a way that the interlocking connection is eliminated when severing the securing part (19).

9. A closure system (1) according to claim 8,
**characterized in that**
the securing part (19) and the securing ring (18) are connected to each other via at least one connecting means (20) which is formed in the longitudinal direction, and the securing part (19) comprises a surface (39) which faces the securing ring (18) and acts as a stop surface in the longitudinal direction for at least one at least ring-segment-shaped elastic securing projection (29) in the first functional position of the closure element (15), said projection being formed on the closure element (15) or the receiving part (30) in the circumferential direction around them.

10. A closure system (1) according to one of the claims 1 to 9,
**characterized in that**
the closure element (57) comprises means (46) for the interlocking and/or friction-locked coupling to the container (4).

11. A closure system (1) according to one of the claims 1 to 10,
**characterized in that**
the applicator projection (3) is formed in a hollow-cylindrical manner and comprises at least one conically formed section, wherein the applicator projection (1) is formed by a region integrally attached to the container (4), and the securing ring (18) is fastened to the container (4) directly or via a holding ring in the circumferential direction and in the direction of the longitudinal axis (AL), said holding ring surrounding the applicator projection (3) in the circumferential direction, or that the applicator projection (3) is formed in a hollow-cylindrical manner and comprises at least one conically formed section and the applicator projection (3) is formed by a separate element or is co-formed on a component of the closure system (1) which is connected at least indirectly in an interlocking and/or friction-locked manner to the container (4).

12. A closure system (1) according to claim 11,
**characterized in that**
it comprises a sealing plug (23) which can be introduced into an opening (6) at the end side on the container (4), comprises a through-opening (40) for connection between the interior space of the container and the ambient environment, and has a circumferential surface which extends on the exterior relating to the longitudinal axis (AL) and which forms a sealing surface over a section for resting on a section of the inner circumference of the container (4) in the region of the opening (6) at the end, the sealing plug is fixed in a friction-locked and/or interlocking manner to the container (4) via a covering cap (9) which surrounds said plug, and the applicator projection (3) is formed by at least one section of the covering cap (9) or by a section on the sealing plug (23) extending through the covering cap (9).

13. A closure system (1) according to claim 12,
**characterized in that**
the covering cap (9) is formed as a hollow-cylindrical element, comprising at least two sections arranged coaxially with respect to each other, a first section (44) for forming the connecting region of the applicator projection (3), a second section (45) for fixing to the container (4), and optionally a third section (47) for the arrangement of connecting means for the indirect friction-locked and/or interlocking connection (22, 51) of the closure element (15) with a connecting part when the covering cap (9) is formed as the connecting part, wherein the optionally provided third section surrounds the first section at least partly over its extension in the direction of the longitudinal axis (AL) in the circumferential direction by forming an intermediate space, and/or the connecting element (57) is formed by the covering cap (9) which in the connecting region to the container (4) comprises latching means which are effective in relation to said container.

14. A closure system (1) according to one of the preceding claims,
**characterized in that**
the closure securing unit (17), in a relative movement in the longitudinal direction in relation to the connecting element (57) from a first to a second mounting position, is formed to at least partly fix the sleeve-like connecting element (57) to the container (4).

15. A closure system (1) according to claim 14,
**characterized in that**
a clamping ring (58) is provided, which surrounds the securing ring (18) of the closure securing device (17) in the second mounting position in the circumferential direction in the region of extension of the connecting element (57) and which clamps the securing ring and the connecting element (57) in relation to the container (4).

16. A closure system (1) according to one of the preceding claims,
**characterized in that**
it is formed with the applicator projection (3) as a pre-assembled structural unit which maintains the closure position between the closure element (15) and the applicator projection (3).

17. The use of a closure system (1) according to one of the claims 1 to 16 for closing at least one container (4), especially a double-chamber container, which receives at least one pharmaceutical preparation.

18. A container (4) for receiving pharmaceutical preparations, comprising an applicator projection (3) which is formed thereon or fixed thereto and comprises a closure system (1) according to one of the claims 1 to 16.

19. A container (4) according to claim 18,
**characterized in that**
it is formed as a double-chamber container with a mouthpiece (8) for forming the opening (6) and for connecting the applicator projection (3), wherein the mouthpiece (8) comprises at least two means (46) which are formed in a complementary manner in relation to the means on the connecting element (57) in order to realise a friction-locked connection and/or an interlocking connection, which are arranged in an offset manner in relation to each other in the longitudinal direction in order to retain the connecting element (57) in a first position in relation to the container (4) in the first mounting position of the closure system (1) and to fix the connecting element (57) in a second position in relation to the container (4) in the second mounting position of the closure system (1).

20. A syringe (5) for medical purposes, comprising a container (4) according to one of the claims 18 or 19.

21. A method for mounting a container (4) with a closure system (1) according to one of the claims 18 or 19,
**characterized by the following features:**
- pre-assembly of the closure system (1) from at least one closure element (15) and closure securing unit (17) to form a structural unit;
- cleaning and/or sterilisation of the closure system (1);
- allocation to a container (4) for receiving pharmaceutical products and closure of an end region (7) of a container (4) receiving pharmaceutical products;
- closure of the container (4) by slipping on the closure system (1).

22. A method according to claim 21,
**characterized in that**
- the closure system (1) is brought to the container prior to lyophilisation to a first mounting position in which the closure element (57), at the end region (7) of the container (4) having the opening (6), is arranged in a first position surrounding said container in the circumferential direction, and the closure securing unit (17) and the closure element (15) which is coupled thereto is positioned as seen in the longitudinal direction in relation to the connecting element (57) in a position free from sealing of the applicator projection (3), and
- the closure of the container (4) during or after lyophilisation occurs by displacing the closure system (1) to a second mounting position under relative movement of at least individual components of the closure system (1) in relation to each other and/or in relation to the end region (7) containing the opening (6), especially the displacement of the closure securing unit (17) and the closure element (15) relative to the connecting element (57) in the longitudinal direction under sealing closure of the applicator projection (3) and the displacement of the connecting element (57) at the end region (7) of the container under fixing of the closure system (1) to the container (4) after lyophilisation to a second position.

## Revendications

1. Système de fermeture (1) pour un embout de dispositif d'administration (3) qui s'étend le long d'un axe longitudinal théorique (AL), présente une ouverture de passage (2) et est conçu pour se raccorder à un dispositif d'administration sur un récipient (4) pour préparations pharmaceutiques, comprenant
- un élément de fermeture (15) avec au moins une zone présentant une surface d'étanchéité (16) pour fermer hermétiquement l'ouverture de passage (2) de l'embout de dispositif d'administration (3),
- un élément de raccord (57) en forme de douille pour la liaison par friction et/ou par engagement positif au récipient dans la partie d'extrémité du côté du dispositif d'administration,
- une unité de sûreté de fermeture (17) coaxiale par rapport à l'élément de raccord (57) et entourant celui-ci dans le sens de la circonférence avec une bague de sûreté (18) et une partie de sûreté (19) reliée la bague de sûreté (18) par l'intermédiaire de moyens de liaison (20) de façon à pouvoir en être séparée en détruisant irréversiblement ceux-ci, l'unité de sûreté de fermeture (17) étant dimensionnée pour entourer au moins la partie d'extrémité de l'embout de dispositif d'administration (3) et l'élément de fermeture (15) dans le sens de la circonférence autour de l'axe longitudinal (AL) sur une partie de l'étendue longitudinale et pour fixer l'élément de raccord (57) en forme de douille au récipient (4) par un mouvement relatif dans le sens longitudinal par rapport à l'élément de raccord (57) d'une première position de montage à une deuxième,
**caractérisé en ce que**
l'élément de fermeture (15) est relié au moins indirectement de façon amovible, par friction et/ou engagement positif, à une partie de raccord formée par l'embout de dispositif d'administration (3) ou à un élément fonctionnel portant celui-ci ou à la bague de sûreté (18), l'élément de fermeture (15) fermant l'ouverture de passage (2) de l'embout de dispositif d'administration (3) de façon étanche à la pression, aux fluides et au micro-organismes dans une première position fonctionnelle définie comme la position de fermeture, et la liaison par engagement positif et/ou friction (22, 51) étant disposée et réalisée de manière à permettre, après que la partie de sûreté (19) a été séparée de la bague de sûreté (18), un mouvement relatif entre les composants de la liaison (22, 51), en particulier l'élément de fermeture (15) et la partie de raccord, avec une composante directionnelle dans la direction de l'axe longitudinal (AL), de la première position fonctionnelle (position de fermeture) à au moins une autre position fonctionnelle (position de protection contre l'injection et/ou de purge d'air) en maintenant au moins partiellement cette liaison (22, 51), l'élément de fermeture (15) supprimant, dans l'au moins une autre position fonctionnelle, l'étanchéité à la pression, aux fluides et aux micro-organismes de l'ouverture de passage (2) de l'embout de dispositif d'administration (3) réalisée par l'élément de fermeture (15) en formant un espace intermédiaire (32) entre l'élément de fermeture (15), l'embout de dispositif d'administration (3) et/ou la partie de raccord.

2. Système de fermeture (1) selon la revendication 1, **caractérisé en ce que** sont prévus des moyens de purge d'air (21) qui sont au moins partiellement intégrés dans la liaison au moins indirecte (22, 51) entre l'élément de fermeture (15) et la partie de raccord et sont disposés et réalisés de manière à pouvoir, au moins dans la deuxième position fonctionnelle de l'élément de fermeture (15), mettre l'ouverture de passage (2) de l'embout de dispositif d'administration (3) en communication avec l'environnement.

3. Système de fermeture (1) selon la revendication 1 ou 2, **caractérisé en ce que** la liaison au moins indirecte (22, 51) entre l'élément de fermeture (15) et la partie de raccord est formée indirectement par une liaison entre une partie de logement (30) entourant l'élément de fermeture (15) au moins dans le sens de la circonférence, vu autour de l'axe longitudinal (AL), et reliée à celui-ci et à la partie de raccord, et la liaison entre l'élément de fermeture (15) et la partie de logement (30) est réalisée comme l'une des liaisons suivantes ou une combinaison de celles-ci :
- liaison par friction
- liaison par engagement positif
- liaison par solidarité de matière
et un engagement positif (31) agissant dans le sens de l'axe longitudinal (AL) et formé au moins indirectement entre l'élément de fermeture (15) et la partie de sûreté (19) est formé entre la partie de logement (30) et la partie de sûreté (19),
ou
**en ce que** la liaison au moins indirecte (22, 51) entre l'élément de fermeture (15) et la partie de raccord est formée par une liaison directe de l'élément de fermeture (15) et de la partie de raccord, et un engagement positif (31) agissant dans le sens de l'axe longitudinal (AL) et formé au moins indirectement entre l'élément de fermeture (15) et la partie de sûreté (19) est formé entre l'élément de fermeture (15) et la partie de sûreté (19).

4. Système de fermeture (1) selon l'une des revendications précédentes, **caractérisé en ce que** la liaison (22, 51) au moins indirecte, autorisant un mouvement relatif entre l'élément de fermeture (15) et l'élément de raccord avec une composante directionnelle dans la direction de l'axe longitudinal (AL), est réalisée au moins sur une partie avec une liaison par engagement positif et/ou par friction agissant dans le sens de l'axe longitudinal (AL) à l'opposé du sens de déplacement pour défaire l'élément de fermeture (15).

5. Système de fermeture (1) selon l'une des revendications précédentes, **caractérisé en ce que** la liaison (22, 51) au moins indirecte, autorisant un mouvement relatif entre l'élément de fermeture (15) et l'élément de raccord avec une composante directionnelle dans la direction de l'axe longitudinal (AL), est formée par un vissage comprenant une partie portant un filetage intérieur sur l'un des composants à assembler ensemble, soit l'élément de fermeture (15) ou la partie de logement (30), soit la partie de raccord, et une partie portant un filetage extérieur sur l'autre des composants à assembler ensemble, soit la partie de raccord, soit l'élément de fermeture (15) ou la partie de logement (30).

6. Système de fermeture (1) selon la revendication 5, **caractérisé en ce qu'**il est prévu au moins un canal de purge d'air s'étendant avec au moins une composante de direction principale parallèlement à l'axe longitudinal (AL), qui est intégré dans le filetage ou formé par l'interruption des spires du filetage en formant des zones sans filet sur au moins un des composants à assembler ensemble, soit l'élément de fermeture (15) ou la partie de logement (30), soit la partie de raccord, la zone sans filet étant disposée et dimensionnée de telle sorte que la somme des zones angulaires sans filet soit comprise entre 5° et 90°, de préférence entre 8° et 60°, en particulier entre 10° et 45° par rapport à la circonférence totale de 360°,
et/ou
l'autre position fonctionnelle (position de purge d'air) entre l'élément de fermeture (15) et l'élément de raccord est définie en fonction de la géométrie et/ou du dimensionnement du filetage et en particulier du pas du filetage.

7. Système de fermeture (1) selon l'une des revendications 1 à 4, **caractérisé en ce que** la liaison (22, 51) au moins indirecte, autorisant un mouvement relatif entre l'élément de fermeture (15) et l'élément de raccord avec une composante directionnelle dans la direction de l'axe longitudinal (AL) est réalisée comme une liaison par engagement positif, comprenant des saillies disposées sur l'un des composants à assembler ensemble, soit l'élément de fermeture (15) ou la partie de logement (30), soit la partie de raccord, et dirigées vers l'autre composant, qui peuvent être introduites sur l'autre des composants à assembler ensemble, soit la partie de raccord ou l'élément de fermeture (15), soit la partie de logement (30), dans des découpes (52) ouvertes sur le bord, s'étendant dans le sens de de l'axe longitudinal et débouchant chacune dans une partie orientée dans le sens de la circonférence.

8. Système de fermeture (1) selon l'une des revendications précédentes, **caractérisé en ce que** dans la première position fonctionnelle, il est prévu entre l'élément de fermeture (15) et la partie de sûreté (19) un engagement positif (31) efficace au moins dans un sens dans la direction de l'axe longitudinal (AL) et l'engagement positif (31) efficace dans la direction de l'axe longitudinal (AL) et formé au moins indirectement entre l'élément de fermeture (25) et la partie de sûreté (19) est disposé dans la zone de liaison (28) entre la partie de sûreté (19) et la bague de sûreté (18) et réalisé de telle manière que l'engagement positif soit aboli quand la partie de sûreté (19) est détachée.

9. Système de fermeture (1) selon la revendication 8, **caractérisé en ce que** la partie de sûreté (19) et la bague de sûreté (18) sont reliées entre elles par au moins un moyen de liaison (20) formé dans le sens longitudinal et la partie de sûreté (19) présente une surface (39) dirigée vers la bague de sûreté (18), qui sert de surface de butée dans le sens longitudinal à au moins une saillie de sûreté élastique (29) en forme au moins de segment d'anneau, formée sur l'élément de fermeture (15) ou la partie de logement (30) en l'entourant dans le sens de la circonférence, dans la position fonctionnelle de l'élément de fermeture (15).

10. Système de fermeture (1) selon l'une des revendications 1 à 9, **caractérisé en ce que** l'élément de raccord (57) présente des moyens (46) pour un couplage par engagement positif et/ou par friction avec le récipient (4).

11. Système de fermeture (1) selon l'une des revendications 1 à 10, **caractérisé en ce que** l'embout de dispositif d'administration (3) est réalisé en forme de cylindre creux et présente au moins une partie conique, l'embout de dispositif d'administration (1) étant formé par une zone moulée sur le récipient (4) et la bague de sûreté (18) étant fixée sur le récipient (4) dans le sens de la circonférence et dans le sens de l'axe longitudinal (AL), directement ou par l'intermédiaire d'une bague de maintien entourant l'embout de dispositif d'administration (3) dans le sens de la circonférence, ou **en ce que** l'embout de dispositif d'administration (3) est réalisé en forme de cylindre creux et présente au moins une partie conique et
l'embout de dispositif d'administration (3) est formé par un élément séparé ou formé solidaire d'un composant du système de fermeture (1) qui est relié au moins indirectement par engagement positif et/ou par friction avec le récipient (4).

12. Système de fermeture (1) selon la revendication 11, **caractérisé en ce qu'**il possède un bouchon d'obturation (23) pouvant être inséré dans une ouverture d'extrémité (6) du récipient (4) et présentant une ouverture de passage (40) pour mettre en communication l'intérieur du récipient et l'environnement, avec une surface de circonférence extérieure s'étendant par rapport à l'axe longitudinal (AL), qui forme dans une partie une surface d'étanchéité destinée à s'appuyer sur une partie de la circonférence intérieure du récipient (4) au niveau de l'ouverture d'extrémité (6),
le bouchon d'obturation est fixé sur le récipient (4) par friction et/ou engagement positif par un clapet de recouvrement (9) qui l'entoure et
l'embout de dispositif d'administration (3) est formé par au moins une partie du clapet de recouvrement (9) ou une partie du bouchon d'obturation (23) s'étendant à travers le clapet de recouvrement (9).

13. Système de fermeture (1) selon la revendication 12, **caractérisé en ce que** le clapet de recouvrement (9) est conçu comme un élément cylindrique creux, comprenant au moins deux parties coaxiales l'une par rapport à l'autre, une première partie (44) pour former la zone de raccordement de l'embout de dispositif d'administration (3), une deuxième partie (45) pour la fixation au récipient (4), et facultativement une troisième partie (47) pour disposer des moyens de liaison en vue de la liaison indirecte par friction et/ou correspondance de forme (22, 51) de l'élément de fermeture (15) avec une partie de raccord en conformant le clapet de recouvrement (9) comme la partie de raccord, la troisième partie prévue facultativement entourant la première partie au moins en partie sur l'étendue de celle-ci dans la direction de l'axe longitudinal (AL) dans le sens de la circonférence en formant un espace intermédiaire et/ou
**en ce que** l'élément de raccord (57) est formé par le clapet de recouvrement (9), qui présente des moyens d'enclenchement agissant vis-à-vis du récipient (4) dans la zone de raccordement à celui-ci.

14. Système de fermeture (1) selon l'une des revendications précédentes, **caractérisé en ce que** l'unité de sûreté de fermeture (17) est conçue pour fixer au moins partiellement l'élément de raccord (57) en forme de douille sur le récipient (4) lors d'un mouvement relatif dans le sens longitudinal par rapport à l'élément de raccord (57) d'une première position de montage à une deuxième.

15. Système de fermeture (1) selon la revendication 14, **caractérisé en ce qu'**il est prévu une bague de serrage (58) entourant la bague de sûreté (18) de l'unité de sûreté de fermeture (17) dans le sens de la circonférence dans la deuxième position de montage, dans la zone d'étendue de l'élément de raccord (57), qui serre la bague de sûreté et l'élément de raccord (57) vis-à-vis du récipient (4).

16. Système de fermeture (1) selon l'une des revendications précédentes, **caractérisé en ce qu'**il est réalisé avec l'embout de dispositif d'administration (3) formant une unité de construction préassemblée qui maintient la position de fermeture entre l'élément de fermeture (15) et l'embout de dispositif d'administration (3).

17. Utilisation d'un système de fermeture (1) selon l'une des revendications 1 à 16 pour la fermeture d'un récipient (4) contenant au moins une préparation pharmaceutique, en particulier d'un récipient à double chambre.

18. Récipient (4) destiné à contenir des préparations pharmaceutiques avec un embout de dispositif d'administration (3) formé ou fixé dessus et muni d'un système de fermeture (1) selon l'une des revendications 1 à 16.

19. Récipient (4) selon la revendication 18, **caractérisé en ce qu'**il est réalisé comme un récipient à double chambre avec une pièce d'embouchure (8) formant l'ouverture (6) et servant au raccordement de l'embout de dispositif d'administration (3), dans lequel la pièce d'embouchure (8) comprend au moins deux moyens complémentaires des moyens (46) de l'élément de raccord (57) pour réaliser une liaison par friction et/ou correspondance de forme, qui sont décalés l'un par rapport à l'autre dans le sens longitudinal afin de maintenir l'élément de raccord (57) dans une première position par rapport au récipient (4) dans la première position de montage du système de fermeture (1), et pour fixer l'élément de raccord (57) dans une deuxième position par rapport au récipient (4) dans une deuxième position de montage du système de fermeture (1).

20. Seringue (5) pour usages médicaux, comprenant un récipient (4) selon l'une des revendications 18 ou 19.

21. Procédé pour le montage d'un récipient (4) avec un système de fermeture (1) selon l'une des revendications 18 ou 19, **caractérisé en ce qu'**il comprend les opérations suivantes :
- préassemblage du système de fermeture (1) à partir au moins d'un élément de fermeture (15) et d'une unité de sûreté de fermeture (17) pour former une unité de construction ;
- nettoyage et/ou stérilisation du système de fermeture (1) ;
- association avec un récipient (4) destiné à contenir des produits pharmaceutiques et fermeture de la partie d'extrémité (7) d'un récipient (4) contenant des produits pharmaceutiques ;
- fermeture du récipient (4) par mise en place coulissante du système de fermeture (1).

22. Procédé selon la revendication 21, **caractérisé en ce que** :
- le système de fermeture (1) est amené sur le récipient, avant la lyophilisation, dans une première position de montage dans laquelle l'élément de raccord (57) placé sur la partie d'extrémité (7) du récipient (4) comportant l'ouverture (6) entoure celui-ci dans le sens de la circonférence dans une première position et l'unité de fermeture de sûreté (17) et l'élément de fermeture (15) couplé avec elle sont positionnés, vus dans le sens longitudinal par rapport à l'élément de raccord (57), dans une position dans laquelle il n'y a pas de fermeture hermétique de l'embout de dispositif d'administration (3) et
- le récipient (4) est fermé pendant ou après une lyophilisation en amenant le système de fermeture (1) dans une deuxième position de montage par un mouvement relatif d'au moins certains composants du système de fermeture (1) les uns par rapport aux autres et/ou par rapport à la partie d'extrémité (7) comportant l'ouverture (6), en particulier par translation de l'unité de fermeture de sûreté (17) et de l'élément de fermeture (15) par rapport à l'élément de raccord (57) dans le sens longitudinal, en fermant hermétiquement l'embout de dispositif d'administration (3), et par translation de l'élément de raccord (57) sur la partie d'extrémité (7) du récipient en fixant le système de fermeture (1) sur le récipient (4) après la lyophilisation dans une deuxième position.
